(19) Europäisches Patentamt
European Patent Office
Office européen des brevets

(11) EP 1 400 514 B1

(12) **EUROPEAN PATENT SPECIFICATION**

(45) Date of publication and mention of the grant of the patent:
**11.02.2009 Bulletin 2009/07**

(21) Application number: **02738730.7**

(22) Date of filing: **17.06.2002**

(51) Int Cl.:
***C07D 221/10*** *(2006.01)* ***C07D 221/16*** *(2006.01)*
***C09K 11/06*** *(2006.01)* ***H05B 33/14*** *(2006.01)*
***H05B 33/12*** *(2006.01)* ***C07F 15/00*** *(2006.01)*

(86) International application number:
**PCT/JP2002/006001**

(87) International publication number:
**WO 2003/000661 (03.01.2003 Gazette 2003/01)**

(54) **METAL COORDINATION COMPOUND AND ELECTROLUMINESCENCE DEVICE**

METALLKOORDINATIONSVERBINDUNG UND ELEKTROLUMINESZENZVORRICHTUNG

COMPOSE DE COORDINATION METALLIQUE ET DISPOSITIF ELECTROLUMINESCENT

(84) Designated Contracting States:
**AT BE CH CY DE DK ES FI FR GB GR IE IT LI LU MC NL PT SE TR**

(30) Priority: **25.06.2001 JP 2001190662**

(43) Date of publication of application:
**24.03.2004 Bulletin 2004/13**

(73) Proprietor: **CANON KABUSHIKI KAISHA**
**Ohta-ku**
**Tokyo 146-8501 (JP)**

(72) Inventors:
- **TAKIGUCHI, Takao**
  **Setagaya-ku, Tokyo 157-0064 (JP)**
- **TSUBOYAMA, Akira**
  **Sagamihara-shi, Kanagawa 229-0011 (JP)**
- **OKADA, Shinjiro**
  **Isehara-shi, Kanagawa 259-1141 (JP)**
- **KAMATANI, Jun**
  **Kawasaki-shi, Kanagawa 215-0011 (JP)**
- **MIURA, Seishi**
  **Sagamihara-shi, Kanagawa 229-0015 (JP)**
- **MORIYAMA, Takashi**
  **Yokohama-shi, Kanagawa 226-0027 (JP)**
- **IGAWA, Satoshi**
  **Fujisawa-shi, Kanagawa 251-0044 (JP)**
- **FURUGORI, Manabu**
  **Atsugi-shi, Kanagawa 243-0004 (JP)**
- **MIZUTANI, Hidemasa**
  **Sagamihara-shi, Kanagawa 228-0816 (JP)**

(74) Representative: **TBK-Patent**
**Bavariaring 4-6**
**80336 München (DE)**

(56) References cited:
**EP-A- 1 191 612** **EP-A2- 1 211 257**
**WO-A-02/02714** **WO-A-02/15645**
**WO-A1-00/57676** **WO-A1-01/41512**
**WO-A1-02/44189** **JP-A- 2001 181 616**
**JP-A- 2001 181 617** **JP-A- 2002 105 055**
**JP-A- 2002 175 884** **US-A1- 2001 019 782**

- **LAMANSKY S ET AL: "MOLECULARLY DOPED POLYMER LIGHT EMITTING DIODES UTILIZING PHOSPHORESCENT PT(II) AND IR(III) DOPANTS" ORGANIC ELECTRONICS, ELSEVIER, AMSTERDAM, NL, vol. 2, no. 1, March 2001 (2001-03), pages 53-62, XP001100541 ISSN: 1566-1199**
- **BALDO M A ET AL: "Very high-efficiency green organic light-emitting devices based on electrophosphorescence" APPLIED PHYSICS LETTERS, AMERICAN INSTITUTE OF PHYSICS. NEW YORK, US, vol. 75, no. 1, 5 July 1999 (1999-07-05), pages 4-6, XP012023409 ISSN: 0003-6951**
- **DJUROVICH P I ET AL: "IR(III) CYCLOMETALATED COMPLEXES AS EFFICIENT PHOSPHORESCENT EMITTERS IN POLYMER BLEND AND ORGANIC LEDS" POLYMER PREPRINTS, AMERICAN CHEMICAL SOCIETY, US, vol. 41, no. 1, March 2000 (2000-03), pages 770-771, XP001052648 ISSN: 0032-3934**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 400 514 B1

- CORNIOLEY-DEUSCHEL CHRISTINE ET AL.: '15. Complexes with a pincers. 2,6-diphenylpyridine as twofold-deprotonated (C-N-C) terdentate ligand in C,C-trans- and as mono-deprotonated (C-N) chelate ligand in chiral C,C-cis-complexes of platinum(II) and palladium(II)' HELV. CHIM. ACTA vol. 1, 1988, pages 130 - 133, XP002955891
- DEUSCHEL-CORNIOLEY CHRISTINE ET AL.: 'A new type of 'square planar' platinum(II) complex showing helical chirality' J. CHEM. SOC., CHEM. COMMUN. vol. 2, 1990, pages 121 - 122, XP002955892
- MAESTRI MAURO ET AL.: 'Spectroscopic and electrochemical properties of pt(II) complexes with aromatic terdendate (C-N-C) cyclometallating ligands' J. PHOTOCHEM. PHOTOBIOL. A: CHEM vol. 67, 1992, pages 173 - 179, XP002955893
- LAMANSKY SERGEY ET AL.: 'Synthesis and characterization of phosphorescent cyclometalated iridium complexes' INORG. CHEM. vol. 40, 26 March 2001, pages 1704 - 1711, XP002196399
- LAMANSKY SERGEY ET AL.: 'Highly phosphorescent bis-cyclometalated iridium complexes: synthesis, photophysical characterization and use in organic light emitting diodes' J. AM. CHEM. SOC. vol. 123, 09 May 2001, pages 4304 - 4312, XP002955894
- JOLLIET PHILIPPE ET AL.: 'Cyclometalated complexes of palladium(II) and platinum(II): cis-configured homoleptic and heteroleptic compounds with aromatic C-N ligands' INORG. CHEM. vol. 35, 1996, pages 4883 - 4888, XP002955895
- OHSAWA Y. ET AL.: 'Electrochemistry and spectroscopy of ortho-matalated complexes of Ir (III) and Rh(III)' J. PHYS. CHEM. vol. 91, 1987, pages 1047 - 1054, XP002955896

## Description

[TECHNICAL FIELD]

The present invention relates to an electroluminescence device using an organic compound, more particularly to an organic electroluminescence device (hereinafter, referred to as an "organic EL device") using a metal coordination compound as a luminescent material.

[BACKGROUND ART]

An applied research of an organic EL device as a luminescence device of a high-speed responsiveness and a high efficiency, has been energetically conducted. Basic structures thereof are shown in Figures 1(a) and (b) (e.g., Macromol Symp. 125, 1 - 48 (1977)).

As shown in Figure 1, an organic EL device generally has a structure comprising a transparent, electrode 14, a metal electrode 11, and a plurality of organic film layers therebetween on a transparent substrate 15.

In the device of Figure 1(a), the organic layers comprise a luminescence layer 12 and a hole-transporting layer 13. For the transparent electrode 14, ITO, etc., having a large work function are used, for providing a good hole-injection characteristic from the transparent electrode 14 to the hole-transporting layer 13. For the metal electrode 11, a metal, such as aluminum, magnesium or an alloy of these, having a small work function is used for providing a good electron-injection characteristic. These electrodes have a thickness of 50 - 200 nm.

For the luminescence layer 12, aluminum quinolynol complexes (a representative example thereof is Alq3 shown hereinafter), etc., having an electron-transporting characteristic and luminescence characteristic are used. For the hole-transporting layer, biphenyldiamine derivatives (a representative example thereof is α-NPD shown hereinafter), etc., having an electron-donative characteristic are used.

The above-structured device has a rectifying characteristic, and when an electric field is applied between the metal electrode 11 as a cathode and the transparent electrode 14 as an anode, electrons are injected from the metal electrode 11 into the luminescence layer 12 and holes are injected from the transparent electrode 15. The injected holes and electrons are recombined within the luminescence layer 12 to form excitons and produce luminescence. At this time, the hole-transporting layer 13 functions as an electron-blocking layer to increase the recombination efficiency at a boundary between the luminescence layer 12 and hole-transporting layer 13, thereby increasing the luminescence efficiency.

Further, in the structure of Figure 1(b), an electron-transporting layer 16 is disposed between the metal electrode 11 and the luminescence layer 12. By separating the luminescence and the electron and hole-transportation to provide a more effective carrier blocking structure, effective luminescence can be performed. For the electron-transporting layer 16, an electron-transporting material, such as an oxidiazole derivative, is used.

Known luminescence processes used heretofore in organic EL devices include fluorescence and phosphorescence. In a fluorescence luminescence device, fluorescence at the time of the transition from a singlet exciton state to the ground state is produced. On the other hand, in the phosphorescence luminescence device, the transition from a triplet exciton state to the ground state is utilized.

In recent years, devices utilizing phosphorescence providing a luminescence yield higher than those utilizing fluorescence have been studied.

Representative published literature may include:

Article 1: Improved energy transfer in electrophosphorescent device (D.F. O'Brien, et al., Applied Physics Letters, Vol. 74, No. 3, p. 422 (1999)); and
Article 2: Very high-efficiency green organic light-emitting devices based on electrophosphorescence (M.A. Baldo, et al., Applied Physics Letters, Vol. 75, No. 1, p. 4 (1999)).

In these articles, a structure including 4 organic layers devices as shown in Figure 1(c) has been principally used, including, from the anode side, a hole-transporting layer 13, a luminescence layer 12, an exciton diffusion-prevention layer 17 and an electron-transporting layer 16. Materials used therein include carrier-transporting materials and phosphorescent materials, of which the abbreviations are shown below.

Alq3: aluminum-quinolinol complex
α-NPD: N4,N4'-di-naphthalene-1-yl-N4,N4'-diphenyl-biphenyl-4,4'-diamine
CBP: 4,4'-N,N'-dicarbazole-biphenyl
BCP: 2,9-dimethyl-4,7-diphehyl-1,10-phenanthroline
PtOEP: platinum-octaethylporphyrin complex

$Ir(ppy)_3$: iridium-phenylpyrimidine complex

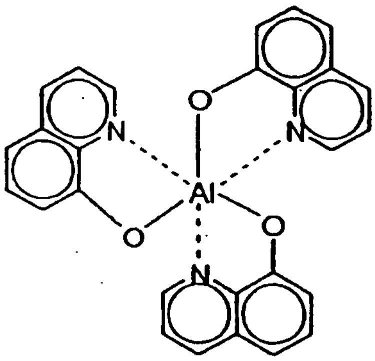

Alq3

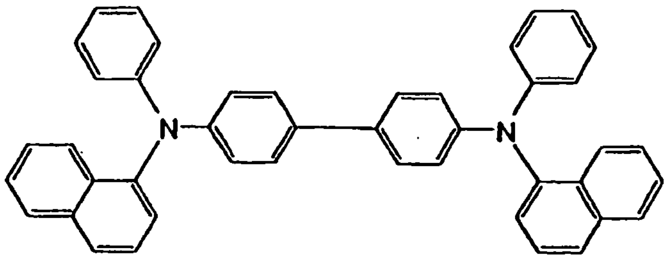

α -NPD

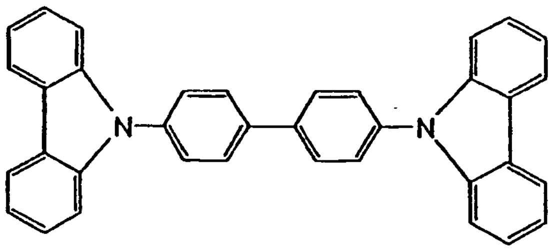

CBP

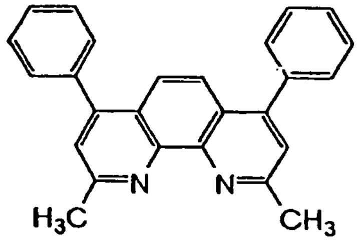

BCP

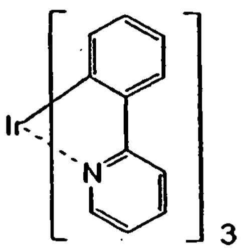

$Ir(ppy)_3$

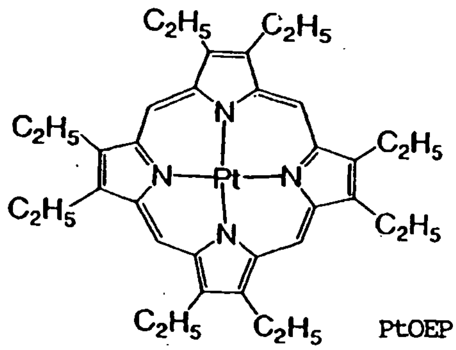

PtOEP

The above-mentioned organic EL devices utilizing phosphorescence have accompanied with a problem of luminescent deterioration particularly in an energized state. The reason for luminescent deterioration has not been clarified as yet but may be attributable to such a phenomenon that the life of triplet exciton is generally longer than that of singlet exciton by at least three digits, so that molecule is placed in a higher-energy state for a long period to cause reaction with ambient substance, formation of exciplex or excimer, change in minute molecular structure, structural change of ambient substance, etc.

Anyway, the phosphorescence luminescence device is expected to provide a higher luminescence efficiency as described above, while the EL device is accompanied with the problem of the luminescent deterioration in energized

state. As a luminescent material used in the phosphorescence device, a compound which produces high-efficiency luminescence and has a high stability is desired.

The article "Synthesis and Characterization of Phosphorescent Cyclometalated Iridium Complexes" by Lamansky et al. (Inorg. Chem. 2001, 40, 1704-1711) reports the preparation, photophysics, and solid state structures of octahedral organometallic Ir complexes with several different cyclometalated ligands. $IrCl_3 \cdot nH_2O$ cleanly cyclometalates a number of different compounds (i.e., 2-phenylpyridine, 2-(*p*-tolyl)pyridine, benzoquinoline, 2-phenylbenzothiazole, 2-(1-naphtyl)benzothiazole, and 2-phenylquinoline), forming the corresponding chloride-bridges dimmers, $C^\wedge N_2$Ir ($\mu$-Cl) $_2$Ir$C^\wedge N_2$ ($C^\wedge N$ is a cyclometalated ligand) in good yield. These chloride-bridged dimmers react with acetyl acetone (acacH) and other bidenate, monoanionic ligands such as picolinic acid (picH) and N-methylsalicylimine (salH), to give monomeric $C^\wedge N_2$Ir (LX) complexes (LX = acac, pic, sal). The emission spectra of these complexes are largely governed by the nature of the cyclometalating ligand, leading to $\lambda_{max}$ values from 510 to 606 nm for the complexes reported here. The strong spin-orbit coupling of iridium mixes the formally forbidden $^3$MLCT and $^3\Pi$-$\Pi^*$ transitions with the allowed $^1$MLCT, leading to a strong phosphorescence with good quantum efficiencies (0.1-0.4) and room temperature lifetimes in the microsecond regime. The emission spectra of the $C^\wedge\Lambda N_2$Ir(LX) complexes are surprisingly similar to the *fac*-Ir$C^\wedge N_3$ complex of the same ligand, even though the structures of the two complexes are markedly different. The crystal structure of two of the $C^\wedge N_2$Ir(acac) complexes (i.e. $C^\wedge N$ = *ppy* and *tpy*) have been determined. Both complexes show *cis-C,C*', *trans*-N,N' disposition of the two cyclometalated ligands, similar to the structures reported for other complexes with a "$C^\wedge N_2$Ir" fragment. NMR data ($^1$H and $^{13}$C) support a similar structure for all of the $C^\wedge N_2$Ir(LX) complexes. Close intermolecular contacts in both $(ppy)_2$Ir(acac) and $(tpy)_2$Ir(acac) lead to significantly red shifted emission spectra for crystalline samples of the *ppy* and *tpy* complexes relative to their solution spectra.

Lamansky et al. describe in "Highly Phosphorescent Bis-Cyclometalated Iridium Complexes: Synthesis, Photophysical Characterization, and Use in Organic Light Emitting Diodes" (J. Am. Chem. Soc. 2001, 123, 4303-4312) the synthesis and photophysical study of a family of cyclometalated iridium(III) complexes. The iridium complexes have two cyclometalated ($C^\wedge N$) ligands and a single monoanionic, bidentate ancillary ligand (LX), i.e. $C^\wedge N_2$Ir(LX). The $C^\wedge N$ ligands can be any of a wide variety of organometallic ligands. The LX ligands used for this study were all $\beta$-diketonates, with the major emphasis placed on acetylacetonate (acac) complexes. The majority of the $C^\wedge N_2$Ir(acac) complexes phosphorescence with high quantum efficiencies (solution quantum yields, 0.1-0.6), and microsecond lifetimes (e.g., 1-14 $\mu$s). The strongly allowed phosphorescence in these complexes is the result of significant spin-orbit coupling of the Ir center. By choosing the appropriate $C^\wedge N$ ligand, $C^\wedge N_2$Ir(acac) complexes can be prepared which emit in any color from green to red. Simple, systematic changes in the $C^\wedge N$ ligands, which lead to bathochromic shifts of the free ligands, lead to similar bathochromic shifts in the Ir complexes of the same ligands, consistent with "$C^\wedge N_2$Ir"-centered emission. Three of the $C^\wedge N_2$Ir(acac) complexes were used as dopants for organic light emitting diodes (OLEDs). The three Ir complexes, i.e., bis(2-phenylpyridinato-N, $C^2$) iridium(acetylacetonate) [$ppy_2$Ir-(acac)], bis(2-phenyl benzothiozolato-N,$C^{2'}$)iridium (acetylaetonate)[$bt_2$Ir(acac)], and bis(2-(2'-benzothienyl)-pyridinato-N, $C^{3'}$)iridium(acetylacetonate) [$btp_2$Ir(acac)], were doped into the emissive region of multilayer, vapor-deposited OLEDs . The $ppy_2$Ir(acac)-, $bt_2$Ir(acac) -, and $btp_2$Ir(acac) - based OLEDs give green yellow, and red electroluminescence, respectively, with very similar current-voltage characteristics.

The publication WO 00/57676 discloses CYCLOMETALLATED METAL COMPLEXES AS PHOSPHORESCENT DOPANTS IN ORGANIC LED'S, wherein the emissive layer of the organic light emitting devices comprises a host material containing an emissive molecule, which molecule is adapted to luminescence when a voltage is applied across the heterostructure, and the emissive molecule is selected from the group of phosphorescent organometallic complexes, including cyclometalated platinum complexes.

The article "Cyclometalated Complexes of Palladium(II) and Platinum(II): cis-Configured Homoleptic and Heteroleptic Compounds with Aromatic C□N Ligands" by Jolliet et al. (Inorg. Chem. 1996, 35, 4883-4888) reports that palladium(II) and platinum(II) bis-homoleptic complexes $M(C□N)_3$, where C□N is benzo(*h*)quinoline (H-bhq), 2-phenylpyridine (H-phpy), 2-(2'-benzothienyl)pyridine (H-bthpy), 2-(2'-thienyl)quinoline (H-thq), and 2-(2'-thienyl)pyridine (H-thpy), were prepared by metal exchange of the lithiated ligands C□N with $M(Et_2S)_2Cl$. The palladium(II) bis-heteroleptic complexes, Pd(C□N) (C'□N'), were synthesized from $Pd(C□N)_2$ bis-homoleptic complexes, which were converted by HCl into the dichloro-bridged compounds $[Pd(C□N)Cl]_2$. By addition of $Et_2S$, the $Pd(C□N)(Et_2S)Cl$ complexes were formed, which were allowed to react with Li(C'□N'), yielding M(C□N)-(C'□N') compounds. An alternative way for obtaining the bis-heteroleptic molecules is by ligand exchange, according to the equilibrium $M(C□N)_2 + M(C'□N')_2$ = 2M (C□N) (C'□N'). The crystal structures of $Pt(bhq)_2$ (**1**) and $Pt(thq)_2$ (**3**) present an important distortion of the square planar (SP-**4**) geometry toward a two-bladed helix. Bis-homoleptic and some bis-heteroleptic complexes of palladium(II) have also been synthesized. In both cases, the steric interactions between the two ligands cause again a helical distortion rather than yielding *rrans* compounds.

Ohsawa et al. describe in "Electrochemistry and Spectroscopy of Ortho-Metalated Complexes of Ir(III) and Rh (III)" (J. Phys. Chem. 1987, 91, 1047-1054) the studies on the electrochemical and UV-visible spectroscopic properties of Rh(III) and Ir(III) complexes of the ortho-metalating (NC) ligands, 2-phenylpyridine (ppy) and benzo[h]quinine (bzq).

Cyclic voltammetric studies of several of the dimeric species, $[M(NC)_2Cl]_2$, indicate metal-centered oxidation occurs at moderate potentials. Cationic monomers of the type $M(NC)_2(NN)^+$ where (NN) = 2,2'-bipyridine or 1,10-phenanthronline have been prepared by reaction of the chelating ligands with the parent dimers. Cyclic voltammetric studies of these monomers indicate that several reversible ligand-centered reductions are generally observed and that the chelating ligand is more easily reduced than is the ortho-metalating ligand. Spectroscopic studies of the mixed ligand monomers indicate that dual emissions from MLCT states associated with the ortho-metalating and chelating ligands occur in the Ir(III) complexes whereas a single emission from a ligand-localized excited state is observed in the Rh(III) complexes. These results are discussed in terms of electronic and nuclear coupling factors analogues to those encountered in descriptions of bimolecular energy and electron-transfer processes.

The Japanese patent application JP 2001-181617 discloses a LIGHT EMITTING ELEMENT MATERIAL COMPOSED OF ORTHOMETALATED PALLADIUM COMPLEX AND LIGHT EMITTING ELEMENT in order to obtain both a light emitting element having excellent luminescence property and a light emitting element material enabling the excellent luminescence proprety. Therefore, the light emitting element material comprises an orthometalated palladium complex having a partial structure of formula (1), formula (2), formula (3) or their tautomers.

[DISCLOSURE OF INVENTION]

Accordingly, an object of the present invention is to provide a stable luminescence device capable of producing high-efficiency luminescence and maintaining a high luminance (brightness) for a long period of time. As a novel luminescent material therefor, the present invention provides a specific metal coordination compound.

The metal coordination compound according to the present invention is represented by the following formula (1):

$$ML_mL'_n \quad (1),$$

wherein M is a metal atom of Ir; L and L' are mutually different bidentate ligands; m is 2 or 3; n is 0, 1 or 2 with the proviso that m+n is 3; a partial structure $ML_m$ is represented by formula (2) shown below and a partial structure $ML'_n$ is represented by formula (3), (4) or (5) shown below:

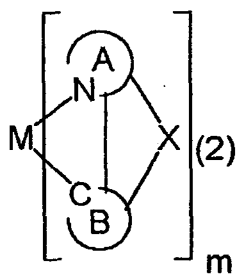 (2) 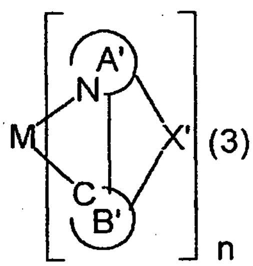 (3) 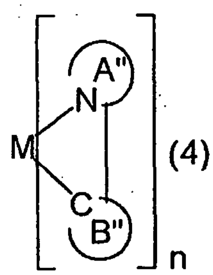 (4) 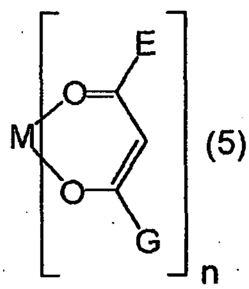 (5)

wherein N and C are nitrogen and carbon atoms, respectively; A and A' are respectively a cyclic group capable of having a substituent and bonded to the metal atom M via the nitrogen atom; B, B' and B" are respectively a cyclic group capable of having a substituent and connected to the metal atom M via the carbon atom;

{wherein the substituent is selected from a halogen atom, a cyano group, a nitro group, a trialkylsilyl group (of which the alkyl groups are independently a linear or branched alkyl group having 1 to 8 carbon atoms), a linear or branched alkyl group having 1 to 20 carbon atoms (of which the alkyl group can include one or non-neighboring two or more methylene groups that can be replaced with -O-, -S-, -CO-, -CO-O-, -O-CO-, -CH=CH- or -C≡C- and the alkyl group can include a hydrogen atom that can be replaced with a fluorine atom), or an aromatic cyclic group capable of having a substituent (of which the substituent is a halogen atom, a cyano group, a nitro group, or a linear or branched alkyl group having 1 to 20 carbon atoms (of which the alkyl group can include one or non-neighboring two or more methylene groups that can be replaced with -O-, -S-, -CO-, -CO-O-, -O-CO-, -CH=CH- or -C≡C- and the alkyl group can include a hydrogen atom that can be replaced with a fluorine atom)}:

A and B, A' and B', and A" and B" are respectively bonded to each other via a covalent bond; and A and B, and A' and B' are respectively bonded to each other via X and X', respectively;

X and X' are independently a linear or branched alkylene group having 2 to 10 carbon atoms (of which the alkylene group can include one or non-neighboring two or more methylene groups that can be replaced with -O-, -S-, -CO-, -CO-O-, -O-CO-, -CH=CH- or -C≡C- and the alkylene group can include a hydrogen atom that

can be replaced with a fluorine atom);
E and G are independently a linear or branched alkyl group having 1 to 20 carbon atoms (of which the alkyl group can include a hydrogen atom that can be optionally replaced with a fluorine atom), or an aromatic cyclic group capable of having a substituent (of which the substituent is a halogen atom, a cyano group, a nitro group, a trialkylsilyl group (of which the alkyl groups are independently a linear or branched alkyl group having 1 to 8 carbon atoms), or a linear or branched alkyl group having 1 to 20 carbon atoms (of which the alkyl group can include one or non-neighboring two or more methylene groups that can be replaced with -O-, -S-, -CO-, -COO-, -O-CO-, -CH=CH- or -C≡C- and the alkyl group can include a hydrogen atom that can be replaced with a fluorine atom)}, wherein the metal coordination compound produces green phosporescence.

The metal coordination compound of the present invention may preferably be one wherein: n is 0 in the formula (1); the partial structure ML'n in the formula (1) in represented by the formula (3); the partial structure ML'n in the formula (1) is represented by the formula (4); or the partial structure ML'n in the formula (1) is represented by the formula (5).

Further, in the formula (1), X may preferably be a linear or branched alkylene group having 2 to 6 carbon atoms (of which the alkylene group can include one or non-neighboring two or more methylene groups that can be replaced with -O-, -S-, -CO-, -CO-O-, -O-CO-, -CH=CH- or -C≡C- and the alkylene group can include a hydrogen atom that can be replaced with a fluorine atom).

Further, the present invention provides an electroluminescence device wherein a layer containing the aforementioned metal coordination compound is sandwiched between two oppositely disposed electrodes and a voltage is applied between the electrodes to produce luminescence.

Particularly, the electroluminescence device may preferably be an electroluminescence device which produces phosphorescence.

[BRIEF DESCRIPTION OF THE DRAWINGS]

Figure 1 illustrates embodiments of the luminescence device according to the present invention, wherein (a) is device structure having two organic layers, (b) is a device structure having three organic layers, and (c) is a device structure having four organic layers.
Figure 2 schematically illustrates an example of a panel structure including an organic EL device and drive means.
Figure 3 illustrates an example of pixel circuit using TFTs (thin film transistors).

[BEST MODE FOR PRACTICING THE INVENTION]

In the case where a luminescence layer is formed of a carrier transporting host material and a phosphorescent guest material, in order to improve a luminescence efficiency of the resultant organic EL device, a luminescence center material per se is required to provide a higher yield of luminescence quantum. In addition thereto, an efficient energy transfer between host material molecules or between host material molecule and guest material molecule is also an important factor.

Further, the reason of the luminescent deterioration has not been clarified as yet but may presumably relate to at least the luminescent material per se or an environmental change thereof by its ambient molecular structure.

For this reason, the present inventors have conducted various studies and have found out the metal coordination compound represented by the above-mentioned formula (1) and have found that an organic EL device using the luminescent material allows a high-efficiency luminescence,keeps a high brightness (luminance) for a long period, and provides less (luminescent) deterioration in energized state.

In the metal coordination compound of formula (1), n may preferably be 0 or 1, more preferably be 0. Further, the partial structure ML'n may preferably be represented by the above-mentioned formula (3).

Further, in the formula (1), X may preferably be a linear or branched alkylene group having 2 to 10 carbon atoms (of which the alkylene group can include one or non-neighboring two or more methylene groups that can be replaced with -O-, -S-, -CO-, -CO-O-, -O-CO-, -CH=CH- or -C≡C- and the alkylene group can include a hydrogen atom that can be replaced with a fluorine atom). Further, in the formula, M may preferably be Ir or Rh, more preferably be Ir.

The metal coordination compound used in the present invention emits phosphorescence, and its lowest excited state is believed to be an MLCT* (metal-to-ligand charge transfer) excited state or π-π* excited state in a triplet state, and phosphorescence is produced at the time of transition from such a state to the ground state.

<Measurement methods of physical properties>

Hereinbelow, methods for measurement of physical properties in the present invention will be described.

(1) Method of judgment between phosphorescence and fluorescence

The identification of phosphorescence was effected depending on whether deactivation with oxygen was caused or not. A (sample) compound is dissolved in chloroform and, after aeration with oxygen or with nitrogen, is subjected to photoillumination to compare photo-luminescence. Almost no luminescence attributable to the compound is observed with respect to the solution aerated with oxygen but in contrast thereto, photo-luminescence is confirmed with respect to the solution aerated with nitrogen, thus allowing differentiation therebetween. The phosphorescence of all the compounds of the present invention has been confirmed by this method unless otherwise noted specifically.

(2) Phosphorescence yield used in the present invention may be determined according to the following formula:

$$\Phi(sample)/\Phi(st) = [Sem(sample)/Iabs(sample)]/[Sem(st)/Iabs(st)],$$

wherein Iabs(st) denotes an absorption coefficient at an excitation Wavelength of the standard sample; Sem(st), a luminescence spectral areal intensity when excited at the same wavelength: Iabs(sample), an absorption coefficient at an excitation wavelength of an objective compound,; and Sem(sample), a luminescence spectral areal intensity when excited at the same wavelength.

Phosphorescence yield values described herein are relative values with respect a phosphorescence yield $\Phi$ = 1 of $Ir(ppy)_3$ as a standard sample.

(3) Method of measurement of phosphorescence life.

A (sample) compound is dissolved in chloroform and spin-coated onto a quartz substrate in a thickness of ca. 0.1 μm and is exposed to pulsative nitrogen laser light at an excitation wavelength of 337 nm at room temperature by using a luminescence life meter (made by Hamamatsu Photonics K.K.). After completion of the excitation pulses, the decay time of luminescence intensity is measure.

When an initial luminescence intensity is denoted by $I_0$, a luminescence intensity after t(set) is expressed according to the following formula with reference to a luminescence life τ(sec):

$$I = I_0 \cdot \exp(-t/\tau).$$

The metal coordination compound of the present invention exhibited a high phosphorescence quantum yield of 0.11 to 0.8 and short phosphorescence life of 1 to 40 psec. If the phosphorescence life is long, the number of molecules placed in triplet excited state waiting for luminescence is increased, thus resulting in a problem of lowering in luminescence efficiency particularly at the time of a high-current density. Accordingly, in order to enhance the luminescence efficiency, it is effective to shorter the above-mentioned phosphorescence life. The metal coordination compound invention has a high phosphorescence quantum yield and a relatively short phosphorescence life, and is therefore suitable as a luminescence material for an organic EL device.

Further, as shown in Example 1 described later, by the alkylene group, represented by X of the formula (2), which is a feature of the present invention, rotational vibration in a direction of a dihedral angle between the intermolecular cyclic groups A and B (further between the intramolecular cyclic groups A' and B', by the alkylene group represented by X' in the case here the partial structure ML'n is represented by the formula (3)) is suppressed. For this reason, the metal coordination compound of the present invention provides a decreased path of energy deactivation within molecule at the time of luminescence, thus being considered that high efficiency-luminescence is achieved.

Further, by appropriately selecting a length of the above-mentioned alkylene group, a dihedral angle between the intramolecular cyclic groups A an B and A' and B' is changed to control an emission wavelength, particularly to allow

shifting toward a shorter wavelength.

Also from the viewpoints described above, the metal coordination compound of the present invention is suitable as the luminescent material of the present invention.

Further, as described in Examples appearing hereinafter, in a continuous energization test, it has been clarified that the metal coordination compound of the present invention exhibits an excellent performance in terms of also a stability. By introducing the above-mentioned alkylene group, which is a feature of the present invention, to cause a change in state as to intermolecular interaction, it is possible to control an intermolecular interaction with the host material etc., thus suppressing formation of excited associates leading to thermal deactivation. As a result, a device characteristic is considered to be improved.

<Synthesis of iridium coordination compound>

A synthesis scheme of the metal coordination compound represented by the formula (1) of the present invention will be shown by taking an iridium coordination compound as an example.

Synthesis of iridium coordination compound

$$Ir\ (CH_3COCHCOCH_3)_3 \xrightarrow{3 \times L} Ir\ (L)_3$$

or

$$IrCl_3 \cdot 3H_2O \xrightarrow{2 \times L} [Ir\ (L)_2Cl]_2 \xrightarrow{L'} Ir\ (L)_2L'$$

Hereinbelow, specific structure formulas of the metal coordination compounds used in the present invention will be shown in Tables 1-1 to Tables 1-14. However, these are merely representative examples and the present invention is not limited to these examples.

$L_1$ to $L_{11}$' used for L and L' shown in Tales 1-1 to 1-14 have the structures shown below.

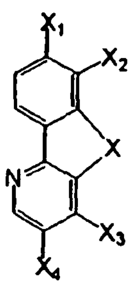
$L_1$

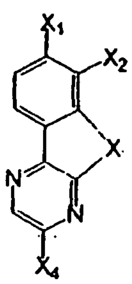
$L_2$

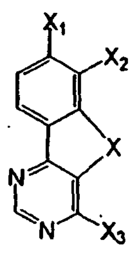
$L_3$

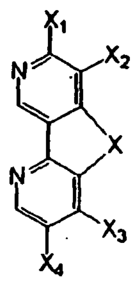
$L_4$

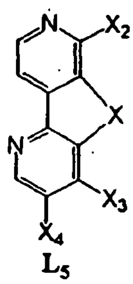
$L_5$

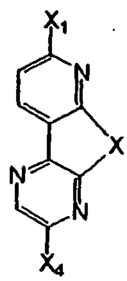
$L_6$

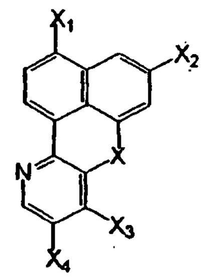

$L_7$

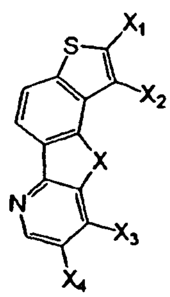

$L_8$

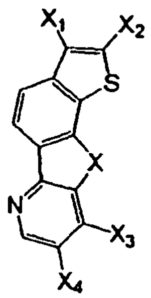

$L_9$

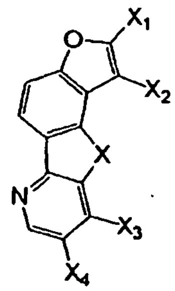

$L_{10}$

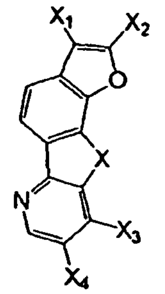

$L_{11}$

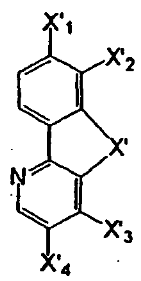

$L_1'$

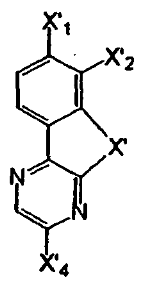

$L_2'$

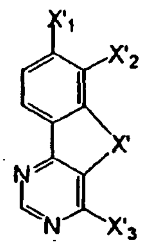

$L_3'$

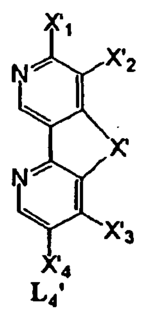

$L_4'$

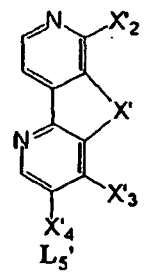

$L_5'$

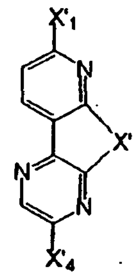

$L_6'$

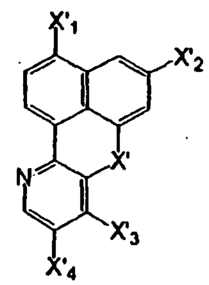

$L_7'$

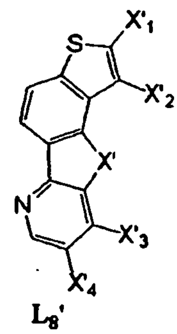

$L_8'$

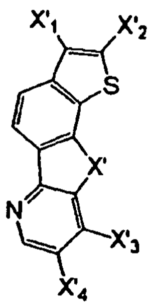

$L_9'$

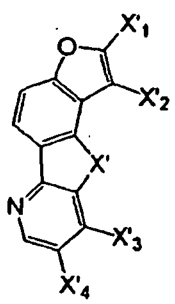

$L_{10}'$

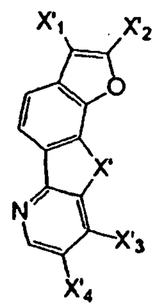

$L_{11}'$

Further, B to M' used for x and X' in Tables 1-1 to 1-14 have the structures shown below.

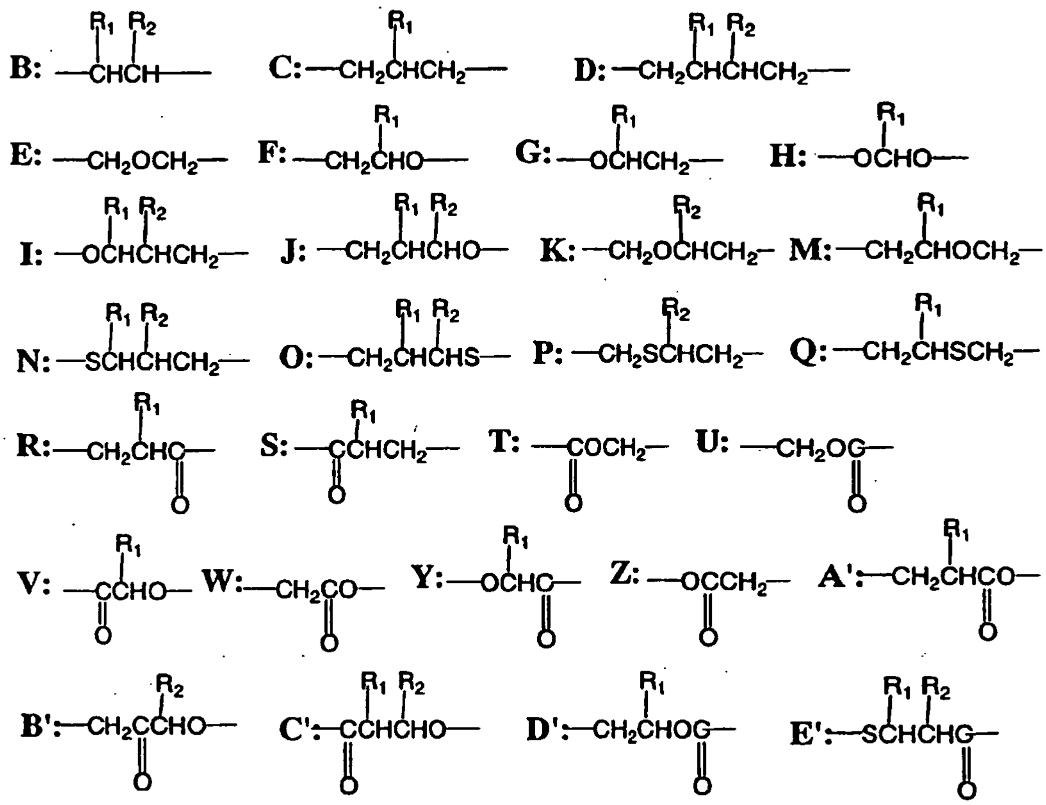

B: C: D: E: F: G: H: I: J: K: M: N: O: P: Q: R: S: T: U: V: W: Y: Z: A': B': C': D': E':

F': $-(CH_2)_5-$ G': $-(CH_2)_6-$ H': $-(CH_2)_7-$ I': $-(CH_2)_8-$

J': $-(CH_2)_9-$ K': $-(CH_2)_{10}-$ M':

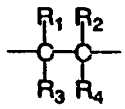

Pi to Qn2 used for cyclic structures A" and B" in Tables 1-1 to 1-11 have the structures shown below.

Pi:

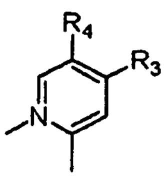

Pr:

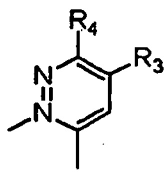

Py1:

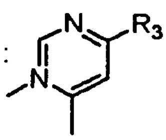

Py2

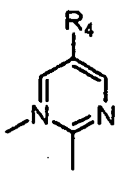

Ph1:

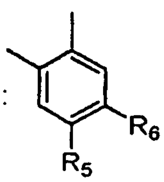

Tn1:

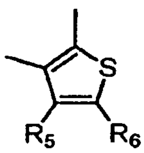

Tn2:

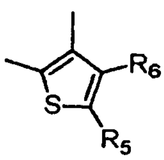

Tn3:

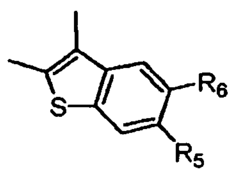

Tn4: 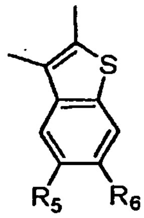 Np1: 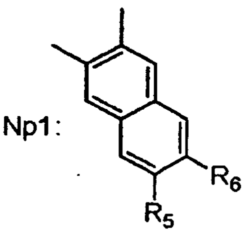 Np2: 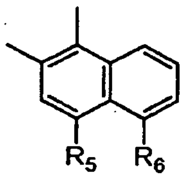 Pe1: 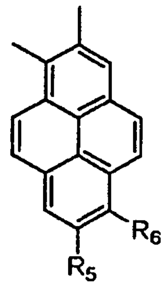

Cn1: 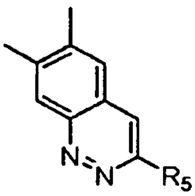 Cn2: 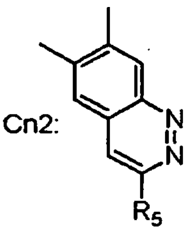 Qn1: 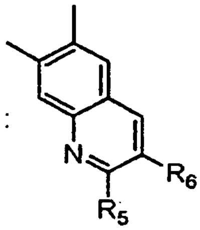 Qn2: 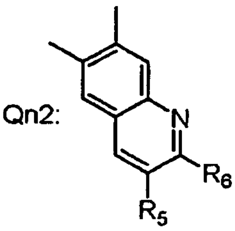

Further, Ph2 to Ph3 used for L and L'; the aromatic groups present as substituents for the cyclic structures A" and B"; and E and G in Tables 1-1 to 1-14 have the structures shown below.

Ph2: 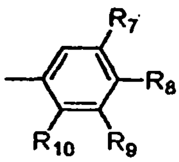 Tn5: 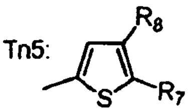 Tn6: 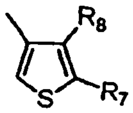 Np3: 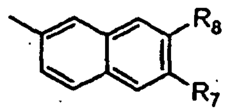

Np4: 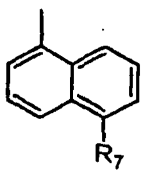 Tn7: 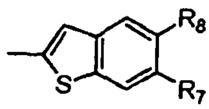 Tn8: 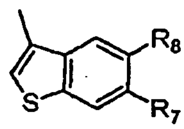

An: 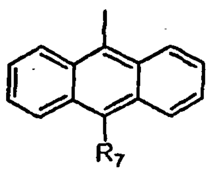 Pe2: 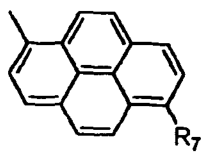 Pi2: 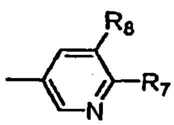 Pi3: 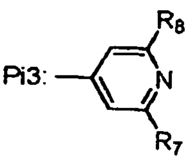

Qn2: 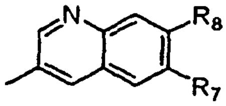 Ph3: 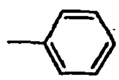

Table 1-1

| No | M | m | n | L | X | R1 | R2 | X1 | X2 | X3 | X4 | R7 | R8 | R9 | R10 |
|---|---|---|---|---|---|---|---|---|---|---|---|---|---|---|---|
| 1 | Ir | 3 | 0 | L1 | B | H | H | H | H | H | H | - | - | - | - |
| 2 | Ir | 3 | 0 | L1 | B | H | H | F | H | H | H | - | - | - | - |
| 3 | Ir | 3 | 0 | L1 | B | H | H | H | F | H | H | - | - | - | - |
| 4 | Ir | 3 | 0 | L1 | B | H | H | F | F | H | H | - | - | - | - |
| 5 | Ir | 3 | 0 | L1 | B | H | H | CF3 | H | H | H | - | - | - | - |
| 6 | Ir | 3 | 0 | L1 | B | H | H | H | CF3 | H | H | - | - | - | - |
| 7 | Ir | 3 | 0 | L1 | B | H | H | F | CF3 | H | H | - | - | - | - |
| 8 | Ir | 3 | 0 | L1 | B | H | H | CF3 | F | H | H | - | - | - | - |
| 9 | Ir | 3 | 0 | L1 | B | H | H | Cl | CF3 | H | H | - | - | - | - |
| 10 | Ir | 3 | 0 | L1 | B | H | H | CH3 | H | H | H | - | - | - | - |
| 11 | Ir | 3 | 0 | L1 | B | H | H | H | CH3 | H | H | - | - | - | - |
| 12 | Ir | 3 | 0 | L1 | B | H | H | OCH3 | H | H | H | - | - | - | - |
| 13 | Ir | 3 | 0 | L1 | B | H | H | H | OCH3 | H | H | - | - | - | - |
| 14 | Ir | 3 | 0 | L1 | B | H | H | OCF3 | H | H | H | - | - | - | - |
| 15 | Ir | 3 | 0 | L1 | B | H | H | H | OCF3 | H | H | - | - | - | - |
| 16 | Ir | 3 | 0 | L1 | B | H | H | Cl | H | H | H | - | - | - | - |
| 17 | Ir | 3 | 0 | L1 | B | H | H | H | Cl | H | H | - | - | - | - |
| 18 | Ir | 3 | 0 | L1 | B | H | H | Br | H | H | H | - | - | - | - |
| 19 | Ir | 3 | 0 | L1 | B | H | H | H | Br | H | H | - | - | - | - |
| 20 | Ir | 3 | 0 | L1 | B | H | H | H | OC4H9 | H | H | - | - | - | - |
| 21 | Ir | 3 | 0 | L1 | B | H | H | OC4H9 | H | H | H | - | - | - | - |
| 22 | Ir | 3 | 0 | L1 | B | H | H | H | OCH (CH3)2 | H | H | - | - | - | - |
| 23 | Ir | 3 | 0 | L1 | B | H | H | Br | H | H | H | - | - | - | - |
| 24 | Ir | 3 | 0 | L1 | B | H | H | H | H | Cl | H | - | - | - | - |
| 25 | Ir | 3 | 0 | L1 | B | H | H | H | H | H | Cl | - | - | - | - |
| 26 | Ir | 3 | 0 | L1 | B | H | H | H | H | CF3 | H | - | - | - | - |
| 27 | Ir | 3 | 0 | L1 | B | H | H | H | H | H | CF3 | - | - | - | - |
| 28 | Ir | 3 | 0 | L1 | B | H | H | Ph3 | H | H | H | - | - | - | - |
| 29 | Ir | 3 | 0 | L1 | B | H | H | Ph3 | H | H | CF3 | - | - | - | - |
| 30 | Ir | 3 | 0 | L1 | B | H | H | Ph2 | H | H | H | H | F | H | H |
| 31 | Ir | 3 | 0 | L1 | B | H | H | Ph2 | H | H | H | H | H | CF3 | H |
| 32 | Ir | 3 | 0 | L1 | B | H | H | Tn5 | H | H | H | H | H | - | - |
| 33 | Ir | 3 | 0 | L1 | B | H | H | Np3 | H | H | H | H | H | - | - |
| 34 | Ir | 3 | 0 | L1 | B | H | H | H | Tn5 | H | H | H | H | - | - |
| 35 | Ir | 3 | 0 | L1 | B | H | H | Tn7 | H | H | H | H | H | - | - |
| 36 | Ir | 3 | 0 | L1 | B | H | H | Pe2 | H | H | H | H | - | - | - |

(continued)

| No | M | m | n | L | X | R1 | R2 | X1 | X2 | X3 | X4 | R7 | R8 | R9 | R10 |
|---|---|---|---|---|---|---|---|---|---|---|---|---|---|---|---|
| 37 | Ir | 3 | 0 | L1 | B | H | H | Tn8 | H | H | H | H | H | - | - |
| 38 | Ir | 3 | 0 | L1 | B | H | H | Np4 | H | H | H | H | - | - | - |
| 39 | Ir | 3 | 0 | L1 | B | H | H | Tn6 | H | H | H | H | H | - | - |
| 40 | Ir | 3 | 0 | L1 | B | CH3 | H | H | H | H | H | - | - | - | - |
| 41 | Ir | 3 | 0 | L1 | B | CH3 | H | F | H | H | H | - | - | - | - |
| 42 | Ir | 3 | 0 | L1 | B | CH3 | H | CF3 | H | H | H | - | - | - | - |
| 43 | Ir | 3 | 0 | L1 | B | CH3 | H | H | CF3 | H | H | - | - | - | - |
| 44 | Ir | 3 | 0 | L1 | B | CH3 | H | F | CF3 | H | H | - | - | - | - |
| 45 | Ir | 3 | 0 | L1 | B | H | CH3 | CF3 | F | H | H | - | - | - | - |
| 46 | Ir | 3 | 0 | L1 | B | H | CH3 | Cl | CF3 | H | H | - | - | - | - |
| 47 | Ir | 3 | 0 | L1 | B | H | CH3 | OC4H9 | H | H | H | - | - | - | - |
| 48 | Ir | 3 | 0 | L1 | B | H | CH3 | H | OCH (CH3)2 | H | H | - | - | - | - |
| 49 | Ir | 3 | 0 | L1 | B | H | CH3 | Ph2 | H | H | H | H | F | H | H |
| 50 | Ir | 3 | 0 | L1 | B | H | CH3 | Np3 | H | H | H | H | H | - | - |

Table 1-2

| No | M | m | n | L | X | R1 | R2 | X1 | X2 | X3 | X4 | R7 | R8 | R9 | R10 |
|---|---|---|---|---|---|---|---|---|---|---|---|---|---|---|---|
| 51 | Ir | 3 | 0 | L1 | B | H | CH3 | Tn6 | H | H | H | H | H | - | - |
| 52 | Ir | 3 | 0 | L1 | B | CH3 | CH3 | H | H | H | H | - | - | - | - |
| 53 | Ir | 3 | 0 | L1 | C | H | - | H | H | H | H | - | - | - | - |
| 54 | Ir | 3 | 0 | L1 | C | H | - | F | H | H | H | - | - | - | - |
| 55 | Ir | 3 | 0 | L1 | C | H | - | H | F | H | H | - | - | - | - |
| 56 | Ir | 3 | 0 | L1 | C | H | - | F | F | H | H | - | - | - | - |
| 57 | Ir | 3 | 0 | L1 | C | H | - | CF3 | H | H | H | - | - | - | - |
| 58 | Ir | 3 | 0 | L1 | C | H | - | H | CF3 | H | H | - | - | - | - |
| 59 | Ir | 3 | 0 | L1 | C | H | - | F | CF3 | H | H | - | - | - | - |
| 60 | Ir | 3 | 0 | L1 | C | H | - | CF3 | F | H | H | - | - | - | - |
| 61 | Ir | 3 | 0 | L1 | C | H | - | Cl | CF3 | H | H | - | - | - | - |
| 62 | Ir | 3 | 0 | L1 | C | H | - | CH3 | H | H | H | - | - | - | - |
| 63 | Ir | 3 | 0 | L1 | C | H | - | H | CH3 | H | H | - | - | - | - |
| 64 | Ir | 3 | 0 | L1 | C | H | - | OCH3 | H | H | H | - | - | - | - |
| 65 | Ir | 3 | 0 | L1 | C | H | - | H | OCH3 | H | H | - | - | - | - |
| 66 | Ir | 3 | 0 | L1 | C | H | - | OCF3 | H | H | H | - | - | - | - |
| 67 | Ir | 3 | 0 | L1 | C | H | - | H | OCF3 | H | H | - | - | - | - |
| 68 | Ir | 3 | 0 | L1 | C | H | - | Cl | H | H | H | - | - | - | - |

(continued)

| No | M | m | n | L | X | R1 | R2 | X1 | X2 | X3 | X4 | R7 | R8 | R9 | R10 |
|---|---|---|---|---|---|---|---|---|---|---|---|---|---|---|---|
| 69 | Ir | 3 | 0 | L1 | C | H | - | H | Cl | H | H | - | - | - | - |
| 70 | Ir | 3 | 0 | L1 | C | H | - | Br | H | H | H | - | - | - | - |
| 71 | Ir | 3 | 0 | L1 | C | H | - | H | Br | H | H | - | - | - | - |
| 72 | Ir | 3 | 0 | L1 | C | H | - | H | OC4H9 | H | H | - | - | - | - |
| 73 | Ir | 3 | 0 | L1 | C | H | - | OC4H9 | H | H | H | - | - | - | - |
| 74 | Ir | 3 | 0 | L1 | C | H | - | H | OCH (CH3)2 | H | H | - | - | - | - |
| 75 | Ir | 3 | 0 | L1 | C | H | - | Br | H | H | H | - | - | - | - |
| 76 | Ir | 3 | 0 | L1 | C | H | - | H | H | Cl | H | - | - | - | - |
| 77 | Ir | 3 | 0 | L1 | C | H | - | H | H | H | Cl | - | - | - | - |
| 78 | Ir | 3 | 0 | L1 | C | H | - | H | H | CF3 | H | - | - | - | - |
| 79 | Ir | 3 | 0 | L1 | C | H | - | H | H | H | CF3 | - | - | - | - |
| 80 | Ir | 3 | 0 | L1 | C | H | - | Ph3 | H | H | H | - | - | - | - |
| 81 | Ir | 3 | 0 | L1 | C | H | - | Ph3 | H | H | CF3 | - | - | - | - |
| 82 | Ir | 3 | 0 | L1 | C | H | - | Ph2 | H | H | H | H | F | H | H |
| 83 | Ir | 3 | 0 | L1 | C | H | - | Ph2 | H | H | H | H | H | CF3 | H |
| 84 | Ir | 3 | 0 | L1 | C | H | - | Tn5 | H | H | H | H | H | - | - |
| 85 | Ir | 3 | 0 | L1 | C | H | - | Np3 | H | H | H | H | H | - | - |
| 86 | Ir | 3 | 0 | L1 | C | H | - | H | Tn5 | H | H | H | H | - | - |
| 87 | Ir | 3 | 0 | L1 | C | H | - | Tn7 | H | H | H | H | H | - | - |
| 88 | Ir | 3 | 0 | L1 | C | H | - | Pe2 | H | H | H | H | - | - | - |
| 89 | Ir | 3 | 0 | L1 | C | H | - | Tn8 | H | H | H | H | H | - | - |
| 90 | Ir | 3 | 0 | L1 | C | H | - | Np4 | H | H | H | H | - | - | - |
| 91 | Ir | 3 | 0 | L1 | C | H | - | Tn6 | H | H | H | H | H | - | - |
| 92 | Ir | 3 | 0 | L1 | C | CH3 | - | H | H | H | H | - | - | - | - |
| 93 | Ir | 3 | 0 | L1 | C | CH3 | - | F | H | H | H | - | - | - | - |
| 94 | Ir | 3 | 0 | L1 | C | CH3 - | - | CF3 | H | H | H | - | - | - | - |
| 95 | Ir | 3 | 0 | L1 | C | CH3 | - | H | CF3 | H | H | - | - | - | - |
| 96 | Ir | 3 | 0 | L1 | C | CH3 | - | F | CF3 | H | H | - | - | - | - |
| 97 | Ir | 3 | 0 | L1 | C | CH3 | - | CF3 | F | H | H | - | - | - | - |
| 98 | Ir | 3 | 0 | L1 | C | CH3 | - | Cl | CF3 | H | H | - | - | - | - |
| 99 | Ir | 3 | 0 | L1 | C | CH3 | - | OC4H9 | H | H | H | - | - | - | - |
| 100 | Ir | 3 | 0 | L1 | C | CH3 | - | H | OCH (CH3)2 | H | H | - | - | - | - |

Table 1-3

| No | M | m | n | L | X R1 | | R2 | X1 | X2 | X3 | X4 | R7 | R8 | R9 | R10 |
|---|---|---|---|---|---|---|---|---|---|---|---|---|---|---|---|
| 101 | Rh | 3 | 0 | L1 | B | H | H | H | H | H | H | - | - | - | - |
| 102 | Rh | 3 | 0 | L1 | B | H | H | F | H | H | H | - | - | - | - |
| 103 | Rh | 3 | 0 | L1 | B | H | H | H | F | H | H | - | - | - | - |
| 104 | Rh | 3 | 0 | L1 | B | H | H | F | F | H | H | - | - | - | - |
| 105 | Rh | 3 | 0 | L1 | B | H | H | CF3 | H | H | H | - | - | - | - |
| 106 | Rh | 3 | 0 | L1 | B | H | H | H | CF3 | H | H | - | - | - | - |
| 107 | Rh | 3 | 0 | L1 | B | H | H | F | CF3 | H | H | - | - | - | - |
| 108 | Rh | 3 | 0 | L1 | B | H | H | CF3 | F | H | H | - | - | - | - |
| 109 | Rh | 3 | 0 | L1 | B | H | H | Cl | CF3 | H | H | - | - | - | - |
| 110 | Rh | 3 | 0 | L1 | B | H | H | CH3 | H | H | H | - | - | - | - |
| 111 | Rh | 3 | 0 | L1 | B | H | H | H | CH3 | H | H | - | - | - | - |
| 112 | Rh | 3 | 0 | L1 | B | H | H | OCH3 | H | H | H | - | - | - | - |
| 113 | Rh | 3 | 0 | L1 | B | H | H | H | OCH3 | H | H | - | - | - | - |
| 114 | Rh | 3 | 0 | L1 | B | H | H | OCF3 | H | H | H | - | - | - | - |
| 115 | Rh | 3 | 0 | L1 | B | H | H | H | OCF3 | H | H | - | - | - | - |
| 116 | Rh | 3 | 0 | L1 | B | H | H | Cl | H | H | H | - | - | - | - |
| 117 | Rh | 3 | 0 | L1 | B | H | H | H | Cl | H | H | - | - | - | - |
| 118 | Rh | 3 | 0 | L1 | B | H | H | Br | H | H | H | - | - | - | - |
| 119 | Rh | 3 | 0 | L1 | B | H | H | H | Br | H | H | - | - | - | - |
| 120 | Rh | 3 | 0 | L1 | B | H | H | H | OC4H9 | H | H | - | - | - | - |
| 121 | Rh | 3 | 0 | L1 | B | H | H | OC4H9 | H | H | H | - | - | - | - |
| 122 | Rh | 3 | 0 | L1 | B | H | H | H | OCH (CH3)2 | H | H | - | - | - | - |
| 123 | Rh | 3 | 0 | L1 | B | H | H | Br | H | H | H | - | - | - | - |
| 124 | Rh | 3 | 0 | L1 | B | H | H | H | H | Cl | H | - | - | - | - |
| 125 | Rh | 3 | 0 | L1 | B | H | H | H | H | H | Cl | - | - | - | - |
| 126 | Pt | 2 | 0 | L1 | B | H | H | H | H | CF3 | H | - | - | - | - |
| 127 | Pt | 2 | 0 | L1 | B | H | H | H | H | H | CF3 | - | - | - | - |
| 128 | Pt | 2 | 0 | L1 | B | H | H | Ph3 | H | H | H | - | - | - | - |
| 129 | Pt | 2 | 0 | L1 | B | H | H | Ph3 | H | H | CF3 | - | - | - | - |
| 130 | Pt | 2 | 0 | L1 | B | H | H | Ph2 | H | H | H | H | F | H | H |
| 131 | Pt | 2 | 0 | L1 | B | H | H | Ph2 | H | H | H | H | H | CF3 | H |
| 132 | Pt | 2 | 0 | L1 | B | H | H | Tn5 | H | H | H | H | H | - | - |
| 133 | Pt | 2 | 0 | L1 | B | H | H | Np3 | H | H | H | H | H | - | - |
| 134 | Pt | 2 | 0 | L1 | B | H | H | H | Tn5 | H | H | H | H | - | - |
| 135 | Pt | 2 | 0 | L1 | B | H | H | Tn7 | H | H | H | H | H | - | - |

(continued)

| No | M | m | n | L | X R1 | | R2 | X1 | X2 | X3 | X4 | R7 | R8 | R9 | R10 |
|---|---|---|---|---|---|---|---|---|---|---|---|---|---|---|---|
| 136 | Pt | 2 | 0 | L1 | B | CH3 | H | F | H | H | H | - | - | - | - |
| 137 | Pt | 2 | 0 | L1 | B | CH3 | H | CF3 | H | H | H | - | - | - | - |
| 138 | Pt | 2 | 0 | L1 | B | CH3 | H | H | CF3 | H | H | - | - | - | - |
| 139 | Pt | 2 | 0 | L1 | B | CH3 | H | F | CF3 | H | H | - | - | - | - |
| 140 | Pt | 2 | 0 | L1 | B | CH3 | H | H | H | H | H | - | - | - | - |
| 141 | Pd | 2 | 0 | L1 | B | CH3 | H | F | H | H | H | - | - | - | - |
| 142 | Pd | 2 | 0 | L1 | B | CH3 | H | CF3 | H | H | H | - | - | - | - |
| 143 | Pd | 2 | 0 | L1 | B | CH3 | H | H | CF3 | H | H | - | - | - | - |
| 144 | Pd | 2 | 0 | L1 | B | CH3 | H | F | CF3 | H | H | - | - | - | - |
| 145 | Pd | 2 | 0 | L1 | B | H | CH3 | CF3 | F | H | H | - | - | - | - |
| 146 | Pd | 2 | 0 | L1 | B | H | CH3 | Cl | CF3 | H | H | - | - | - | - |
| 147 | Pd | 2 | 0 | L1 | B | H | CH3 | OC4H9 | H | H | H | - | - | - | - |
| 148 | Pd | 2 | 0 | L1 | B | H | CH3 | H | OCH (CH3)2 | H | H | - | - | - | - |
| 149 | Pd | 2 | 0 | L1 | B | H | CH3 | Ph2 | H | H | H | H | F | H | H |
| 150 | Pd | 2 | 0 | L1 | B | H | CH3 | Np3 | H | H | H | H | H | - | - |

Table 1-4

| No | M | m | n | L X | | R1 | R2 | X1 | X2 | X3 | X4 | R7 | R8 | R9 | R10 |
|---|---|---|---|---|---|---|---|---|---|---|---|---|---|---|---|
| 151 | Ir | 3 | 0 | L1 | D | H | H | H | H | H | H | - | - | - | - |
| 152 | Ir | 3 | 0 | L1 | D | H | H | F | H | H | H | - | - | - | - |
| 153 | Ir | 3 | 0 | L1 | D | H | H | H | F | H | H | - | - | - | - |
| 154 | Ir | 3 | 0 | L1 | D | H | H | F | F | H | H | - | - | - | - |
| 155 | Ir | 3 | 0 | L1 | D | H | H | CF3 | H | H | H | - | - | - | - |
| 156 | Ir | 3 | 0 | L1 | D | H | H | H | CF3 | H | H | - | - | - | - |
| 157 | Ir | 3 | 0 | L1 | D | H | H | F | CF3 | H | H | - | - | - | - |
| 158 | Ir | 3 | 0 | L1 | D | H | H | CF3 | F | H | H | - | - | - | - |
| 159 | Ir | 3 | 0 | L1 | D | H | H | Cl | CF3 | H | H | - | - | - | - |
| 160 | Ir | 3 | 0 | L1 | D | H | H | CH3 | H | H | H | - | - | - | - |
| 161 | Ir | 3 | 0 | L1 | D | H | H | H | CH3 | H | H | - | - | - | - |
| 162 | Ir | 3 | 0 | L1 | D | CH3 | H | OCH3 | H | H | H | - | - | - | - |
| 163 | Ir | 3 | 0 | L1 | D | H | CH3 | H | OCH3 | H | H | - | - | - | - |
| 164 | Ir | 3 | 0 | L1 | D | CH3 | CH3 | OCF3 | H | H | H | - | - | - | - |
| 165 | Ir | 3 | 0 | L1 | D | H | H | H | OCF3 | H | H | - | - | - | - |
| 166 | Ir | 3 | 0 | L1 | E | - | - | H | H | H | H | - | - | - | - |
| 167 | Ir | 3 | 0 | L1 | E | - | - | H | Cl | H | H | - | - | - | - |
| 168 | Ir | 3 | 0 | L1 | E | - | - | Br | H | H | H | - | - | - | - |
| 169 | Ir | 3 | 0 | L1 | E | - | - | H | Br | H | H | - | - | - | - |

(continued)

| No | M | m | n | L X | | R1 | R2 | X1 | X2 | X3 | X4 | R7 | R8 | R9 | R10 |
|---|---|---|---|---|---|---|---|---|---|---|---|---|---|---|---|
| 170 | Ir | 3 | 0 | L1 | E | - | - | H | OC4H9 | H | H | - | - | - | - |
| 171 | Ir | 3 | 0 | L1 | F | H | - | H | H | H | H | - | - | - | - |
| 172 | Ir | 3 | 0 | L1 | F | H | - | H | OCH (CH3)2 | H | H | - | - | - | - |
| 173 | Ir | 3 | 0 | L1 | F | H | - | Br | H | H | H | - | - | - | - |
| 174 | Ir | 3 | 0 | L1 | F | H | - | H | H | Cl | H | - | - | - | - |
| 175 | Ir | 3 | 0 | L1 | F | C2H5 | - | H | H | H | Cl | - | - | - | - |
| 176 | Ir | 3 | 0 | L1 | G | H | - | H | H | CF3 | H | - | - | - | - |
| 177 | Ir | 3 | 0 | L1 | G | H | - | H | H | H | H | - | - | - | - |
| 178 | Ir | 3 | 0 | L1 | G | H | - | Ph3 | H | H | H | - | - | - | - |
| 179 | Ir | 3 | 0 | L1 | G | H | - | Ph3 | H | H | CF3 | - | - | - | - |
| 180 | Ir | 3 | 0 | L1 | G | H | - | H | H | H | H | - | - | - | - |
| 181 | Ir | 3 | 0 | L1 | H | H | - | Ph2 | H | H | H | H | H | CF3 | H |
| 182 | Ir | 3 | 0 | L1 | H | H | - | Tn5 | H | H | H | H | H | - | - |
| 183 | Ir | 3 | 0 | L1 | H | H | - | Np3 | H | H | H | H | H | - | - |
| 184 | Ir | 3 | 0 | L1 | H | CH3 | - | H | Tn5 | H | H | H | H | - | - |
| 185 | Ir | 3 | 0 | L1 | H | H | - | Tn7 | H | H | H | H | H | - | - |
| 186 | Ir | 3 | 0 | L1 | I | H | H | H | H | H | H | - | - | - | - |
| 187 | Ir | 3 | 0 | L1 | I | H | H | Tn8 | H | H | H | H | H | - | - |
| 188 | Ir | 3 | 0 | L1 | I | H | H | Np4 | H | H | H | H | - | - | - |
| 189 | Ir | 3 | 0 | L1 | I | H | H | Tn6 | H | H | H | H | H | - | - |
| 190 | Ir | 3 | 0 | L1 | I | CH3 | H | H | H | H | H | - | - | - | - |
| 191 | Ir | 3 | 0 | L1 | J | H | H | F | H | H | H | - | - | - | - |
| 192 | Ir | 3 | 0 | L1 | J | H | H | CF3 | H | H | H | - | - | - | - |
| 193 | Ir | 3 | 0 | L1 | J | H | H | H | CF3 | H | H | - | - | - | - |
| 194 | Ir | 3 | 0 | L1 | J | CH3 | H | F | CF3 | H | H | - | - | - | - |
| 195 | Ir | 3 | 0 | L1 | J | H | CH3 | CF3 | F | H | H | - | - | - | - |
| 196 | Ir | 3 | 0 | L1 | K | - | H | Cl | CF3 | H | H | - | - | - | - |
| 197 | Ir | 3 | 0 | L1 | K | - | H | OC4H9 | H | H | H | - | - | - | - |
| 198 | Ir | 3 | 0 | L1 | K | - | H | H | OCH (CH3)2 | H | H | - | - | - | - |
| 199 | Ir | 3 | 0 | L1 | K | - | H | Ph2 | H | H | H | H | F | H | H |
| 200 | Ir | 3 | 0 | L1 | K | - | CH3 | Np3 | H | H | H | H | H | - | - |

Table 1-5

| No | M | m | n | L | X | R1 | R2 | X1 | X2 | X3 | X4 | R7 | R8 | R9 | R10 |
|---|---|---|---|---|---|---|---|---|---|---|---|---|---|---|---|
| 201 | Ir | 3 | 0 | L1 | M | H | - | H | H | H | H | - | - | - | - |
| 202 | Ir | 3 | 0 | L1 | M | H | - | F | H | H | H | - | - | - | - |

(continued)

| No | M | m | n | L | X | R1 | R2 | X1 | X2 | X3 | X4 | R7 | R8 | R9 | R10 |
|---|---|---|---|---|---|---|---|---|---|---|---|---|---|---|---|
| 203 | Ir | 3 | 0 | L1 | M | H | - | H | F | H | H | - | - | - | - |
| 204 | Ir | 3 | 0 | L1 | N | H | H | H | H | H | H | - | - | - | - |
| 205 | Ir | 3 | 0 | L1 | N | H | H | CF3 | H | H | H | - | - | - | - |
| 206 | Ir | 3 | 0 | L1 | N | CH3 | H | H | CF3 | H | H | - | - | - | - |
| 207 | Ir | 3 | 0 | L1 | O | H | H | F | CF3 | H | H | - | - | - | - |
| 208 | Ir | 3 | 0 | L1 | O | H | H | CF3 | F | H | H | - | - | - | - |
| 209 | Ir | 3 | 0 | L1 | O | H | H | Cl | CF3 | H | H | - | - | - | - |
| 210 | Ir | 3 | 0 | L1 | P | - | H | H | H | H | H | - | - | - | - |
| 211 | Ir | 3 | 0 | L1 | P | - | H | H | CH3 | H | H | - | - | - | - |
| 212 | Ir | 3 | 0 | L1 | P | - | H | OCH3 | H | H | H | - | - | - | - |
| 213 | Ir | 3 | 0 | L1 | Q | H | - | H | H | H | H | - | - | - | - |
| 214 | Ir | 3 | 0 | L1 | Q | H | - | OCF3 | H | H | H | - | - | - | - |
| 215 | Ir | 3 | 0 | L1 | Q | H | - | H | OCF3 | H | H | - | - | - | - |
| 216 | Ir | 3 | 0 | L1 | R | H | - | H | H | H | H | - | - | - | - |
| 217 | Ir | 3 | 0 | L1 | R | H | - | H | Cl | H | H | - | - | - | - |
| 218 | Ir | 3 | 0 | L1 | R | H | - | H | H | H | H | - | - | - | - |
| 219 | Ir | 3 | 0 | L1 | S | H | - | H | Br | H | H | - | - | - | - |
| 220 | Ir | 3 | 0 | L1 | S | H | - | H | OC4H9 | H | H | - | - | - | - |
| 221 | Ir | 3 | 0 | L1 | S | H | - | OC4H9 | H | H | H | - | - | - | - |
| 222 | Ir | 3 | 0 | L1 | T | - | - | H | H | H | H | - | - | - | - |
| 223 | Ir | 3 | 0 | L1 | T | - | - | Br | H | H | H | - | - | - | - |
| 224 | Ir | 3 | 0 | L1 | T | - | - | H | H | H | H | - | - | - | - |
| 225 | Ir | 3 | 0 | L1 | U | - | - | H | H | H | Cl | - | - | - | - |
| 226 | Ir | 3 | 0 | L1 | U | - | - | H | H | CF3 | H | - | - | - | - |
| 227 | Ir | 3 | 0 | L1 | U | - | - | H | H | H | CF3 | - | - | - | - |
| 228 | Ir | 3 | 0 | L1 | V | H | - | H | H | H | H | - | - | - | - |
| 229 | Ir | 3 | 0 | L1 | V | H | - | Ph3 | H | H | H | - | - | - | - |
| 230 | Ir | 3 | 0 | L1 | V | H | - | Ph2 | H | H | H | H | F | H | H |
| 231 | Ir | 3 | 0 | L1 | W | - | - | H | H | H | H | - | - | - | - |
| 232 | Ir | 3 | 0 | L1 | W | - | - | Tn5 | H | H | H | H | H | - | - |
| 233 | Ir | 3 | 0 | L1 | W | - | - | Np3 | H | H | H | H | H | - | - |
| 234 | Ir | 3 | 0 | L1 | Y | H | - | H | H | H | H | - | - | - | - |
| 235 | Ir | 3 | 0 | L1 | Y | H | - | Tn7 | H | H | H | H | H | - | - |
| 236 | Ir | 3 | 0 | L1 | Y | H | - | Pe2 | H | H | H | H | - | - | - |
| 237 | Ir | 3 | 0 | L1 | Z | - | - | H | H | H | H | - | - | - | - |
| 238 | Ir | 3 | 0 | L1 | Z | - | - | Np4 | H | H | H | H | - | - | - |
| 239 | Ir | 3 | 0 | L1 | Z | - | - | Tn6 | H | H | H | H | H | - | - |
| 240 | Ir | 3 | 0 | L1 | A’ | H | - | H | H | H | H | - | - | - | - |

(continued)

| No | M | m | n | L | X | R1 | R2 | X1 | X2 | X3 | X4 | R7 | R8 | R9 | R10 |
|---|---|---|---|---|---|---|---|---|---|---|---|---|---|---|---|
| 241 | Ir | 3 | 0 | L1 | A’ | H | - | F | H | H | H | - | - | - | - |
| 242 | Ir | 3 | 0 | L1 | A’ | CH3 | - | CF3 | H | H | H | - | - | - | - |
| 243 | Ir | 3 | 0 | L1 | B’ | - | H | H | H | H | H | - | - | - | - |
| 244 | Ir | 3 | 0 | L1 | B’ | - | H | F | CF3 | H | H | - | - | - | - |
| 245 | Ir | 3 | 0 | L1 | B’ | - | CH3 | CF3 | F | H | H | - | - | - | - |
| 246 | Ir | 3 | 0 | L1 | C’ | H | H | Cl | CF3 | H | H | - | - | - | - |
| 247 | Ir | 3 | 0 | L1 | C’ | H | H | OC4H9 | H | H | H | - | - | - | - |
| 248 | Ir | 3 | 0 | L1 | C’ | H | CH3 | H | H | H | H | - | - | - | - |
| 249 | Ir | 3 | 0 | L1 | D’ | H | - | Ph2 | H | H | H | H | F | H | H |
| 250 | Ir | 3 | 0 | L1 | D’ | CH3 | - | Np3 | H | H | H | H | H | - | - |

Table 1-6

| No | M | m | n | L | X | R1 | R2 | X1 | X2 | X3 | X4 | R7 | R8 | R9 | R10 |
|---|---|---|---|---|---|---|---|---|---|---|---|---|---|---|---|
| 251 | Ir | 3 | 0 | L1 | D’ | H | H | H | H | H | H | - | - | - | - |
| 252 | Ir | 3 | 0 | L1 | E’ | H | H | H | H | H | H | - | - | - | - |
| 253 | Ir | 3 | 0 | L1 | E’ | H | H | H | F | H | H | - | - | - | - |
| 254 | Ir | 3 | 0 | L1 | E’ | H | CH3 | F | F | H | H | - | - | - | - |
| 255 | Ir | 3 | 0 | L1 | F’ | - | - | CF3 | H | H | H | - | - | - | - |
| 256 | Ir | 3 | 0 | L1 | F’ | - | - | H | H | H | H | - | - | - | - |
| 257 | Ir | 3 | 0 | L1 | F’ | - | - | F | CF3 | H | H | - | - | - | - |
| 258 | Ir | 3 | 0 | L1 | F’ | - | - | CF3 | F | H | H | - | - | - | - . |
| 259 | Ir | 3 | 0 | L1 | F’ | - | - | Cl | CF3 | H | H | - | - | - | - |
| 260 | Ir | 3 | 0 | L1 | G’ | - | - | H | H | H | H | - | - | - | - |
| 261 | Ir | 3 | 0 | L1 | G’ | - | - | H | CH3 | H | H | - | - | - | - |
| 262 | Ir | 3 | 0 | L1 | G’ | - | - | OCH3 | H | H | H | - | - | - | - |
| 263 | Ir | 3 | 0 | L1 | G’ | - | - | H | OCH3 | H | H | - | - | - | - |
| 264 | Ir | 3 | 0 | L1 | G’ | - | - | OCF3 | H | H | H | - | - | - | - |
| 265 | Ir | 3 | 0 | L1 | G’ | - | - | H | OCF3 | H | H | - | - | - | - |
| 266 | Ir | 3 | 0 | L1 | H’ | - | - | H | H | H | H | - | - | - | - |
| 267 | Ir | 3 | 0 | L1 | H’ | - | - | H | Cl | H | H | - | - | - | - |
| 268 | Ir | 3 | 0 | L1 | H’ | - | - | Br | H | H | H | - | - | - | - |
| 269 | Ir | 3 | 0 | L1 | H’ | - | - | H | Br | H | H | - | - | - | - |
| 270 | Ir | 3 | 0 | L1 | H’ | - | - | H | OC4H9 | H | H | - | - | - | - |
| 271 | Ir | 3 | 0 | L1 | I’ | - | - | H | H | H | H | - | - | - | - |
| 272 | Ir | 3 | 0 | L1 | I’ | - | - | H | OCH (CH3)2 | H | H | - | - | - | - |
| 273 | Ir | 3 | 0 | L1 | I’ | - | - | Br | H | H | H | - | - | - | - |
| 274 | Ir | 3 | 0 | L1 | I’ | - | - | H | H | Cl | H | - | - | - | - |

(continued)

| No | M | m | n | L | X | R1 | R2 | X1 | X2 | X3 | X4 | R7 | R8 | R9 | R10 |
|---|---|---|---|---|---|---|---|---|---|---|---|---|---|---|---|
| 275 | Ir | 3 | 0 | L1 | I' | - | - | H | H | H | Cl | - | - | - | - |
| 276 | Ir | 3 | 0 | L1 | J' | - | - | H | H | H | H | - | - | - | - |
| 277 | Ir | 3 | 0 | L1 | J' | - | - | H | H | H | CF3 | - | - | - | - |
| 278 | Ir | 3 | 0 | L1 | J' | - | - | Ph3 | H | H | H | - | - | - | - |
| 279 | Ir | 3 | 0 | L1 | J' | - | - | Ph3 | H | H | CF3 | - | - | - | - |
| 280 | Ir | 3 | 0 | L1 | J' | - | - | Ph2 | H | H | H | H | F | H | H |
| 281 | Ir | 3 | 0 | L1 | K' | - | - | Ph2 | H | H | H | H | H | CF3 | H |
| 282 | Ir | 3 | 0 | L1 | K' | - | - | H | H | H | H | - | - | - | - |
| 283 | Ir | 3 | 0 | L1 | K' | - | - | Np3 | H | H | H | H | H | - | - |
| 284 | Ir | 3 | 0 | L1 | K' | - | - | H | Tn5 | H | H | H | H | - | - |
| 285 | Ir | 3 | 0 | L1 | K' | - | - | Tn7 | H | H | H | H | H | - | - |
| 286 | Rh | 3 | 0 | L1 | C | H | - | Pe2 | H | H | H | H | - | - | - |
| 287 | Rh | 3 | 0 | L1 | C | H | - | Tn8 | H | H | H | H | H | - | - |
| 288 | Rh | 3 | 0 | L1 | C | H | - | Np4 | H | H | H | H | - | - | - |
| 289 | Rh | 3 | 0 | L1 | I | H | H | Tn6 | H | H | H | H | H | - | - |
| 290 | Rh | 3 | 0 | L1 | D' | CH3 | - | H | H | H | H | - | - | - | - |
| 291 | Rh | 3 | 0 | L1 | F | - | - | F | H | H | H | - | - | - | - |
| 292 | Pt | 2 | 0 | L1 | C | H | - | CF3 | H | H | H | - | - | - | - |
| 293 | Pt | 2 | 0 | L1 | O | H | H | H | CF3 | H | H | - | - | - | - |
| 294 | Pt | 2 | 0 | L1 | Z | - | - | F | CF3 | H | H | - | - | - | - |
| 295 | Pt | 2 | 0 | L1 | D' | H | - | CF3 | F | H | H | - | - | - | - |
| 296 | Pt | 2 | 0 | L1 | F' | - | - | Cl | CF3 | H | H | - | - | - | - |
| 297 | Pt | 2 | 0 | L1 | H' | - | - | OC4H9 | H | H | H | - | - | - | - |
| 298 | Pd | 2 | 0 | L1 | I' | - | - | H | OCH (CH3)2 | H | H | - | - | - | - |
| 299 | Pd | 2 | 0 | L1 | G | H | - | Ph2 | H | H | H | H | F | H | H |
| 300 | Pd | 2 | 0 | L1 | C | H | - | Np3 | H | H | H | H | H | - | - |

Table 1-7

| No | M | m | n | L | X | R1 | R2 | X1 | X2 | X3 | X4 | R7 | R8 | R9 | R10 |
|---|---|---|---|---|---|---|---|---|---|---|---|---|---|---|---|
| | | | | L' | X' | R1 | R2 | X1' | X2' | X3' | X4' | R7 | R8 | R9 | R10 |
| 301 | Ir | 2 | 1 | L1 | B | H | H | H | H | H | H | - | - | - | - |
| | | | | L1' | B | H | H | F | H | H | H | - | - | - | - |
| 302 | Ir | 2 | 1 | L1 | B | H | H | H | H | H | H | - | - | - | - |
| | | | | L1' | B | H | H | H | F | H | H | - | - | - | - |
| 303 | Ir | 2 | 1 | L1 | B | H | H | H | H | H | H | - | - | - | - |
| | | | | L1' | B | H | H | F | F | H | H | - | - | - | - |

(continued)

| No | M | m | n | L / L' | X / X' | R1 | R2 | X1 / X1' | X2 / X2' | X3 / X3' | X4 / X4' | R7 | R8 | R9 | R10 |
|---|---|---|---|---|---|---|---|---|---|---|---|---|---|---|---|
| 304 | Ir | 2 | 1 | L1 | B | H | H | H | H | H | H | - | - | - | - |
| | | | | L1' | B | H | H | CF3 | H | H | H | - | - | - | - |
| 305 | Ir | 2 | 1 | L1 | B | H | H | H | H | H | H | - | - | - | - |
| | | | | L1' | B | H | H | H | CF3 | H | H | - | - | - | - |
| 306 | Ir | 2 | 1 | L1 | B | H | H | H | H | H | H | - | - | - | - |
| | | | | L1' | B | H | H | F | CF3 | H | H | - | - | - | - |
| 307 | Ir | 2 | 1 | L1 | B | H | H | H | H | H | H | - | - | - | - |
| | | | | L1' | B | H | H | Cl | CF3 | H | H | - | - | - | - |
| 308 | Ir | 2 | 1 | L1 | B | H | H | H | H | H | H | - | - | - | - |
| | | | | L1' | B | H | H | CH3 | H | H | H | - | - | - | - |
| 309 | Ir | 2 | 1 | L1 | B | H | H | H | H | H | H | - | - | - | - |
| | | | | L1' | B | H | H | OCF3 | H | H | H | - | - | - | - |
| 310 | Ir | 2 | 1 | L1 | B | H | H | H | H | H | H | - | - | - | - |
| | | | | L1' | B | H | H | H | OC4H9 | H | H | - | - | - | - |
| 311 | Ir | 2 | 1 | L1 | B | H | H | OC4H9 | H | H | H | - | - | - | - |
| | | | | L1' | B | H | H | H | OCH (CH3)2 | H | H | - | - | - | - |
| 312 | Ir | 2 | 1 | L1 | B | H | H | Br | H | H | H | - | - | - | - |
| | | | | L1' | B | H | H | H | H | Cl | H | - | - | - | - |
| 313 | Ir | 2 | 1 | L1 | B | H | H | H | H | H | Cl | - | - | - | - |
| | | | | L1' | B | H | H | H | H | CF3 | H | - | - | - | - |
| 314 | Ir | 2 | 1 | L1 | B | H | H | H | H | H | CF3 | - | - | - | - |
| | | | | L1' | B | H | H | Ph3 | H | H | H | - | - | - | - |
| 315 | Ir | 2 | 1 | L1 | B | H | H | Ph3 | H | H | CF3 | - | - | - | - |
| | | | | L1' | B | H | H | Ph2 | H | H | H | H | F | H | H |
| 316 | Ir | 2 | 1 | L1 | B | H | H | Ph2 | H | H | H | H | H | CF3 | H |
| | | | | L1' | B | H | H | Tn5 | H | H | H | H | H | - | - |
| 317 | Ir | 2 | 1 | L1 | B | H | H | Np3 | H | H | H | H | H | - | - |
| | | | | L1' | B | H | H | H | Tn5 | H | H | H | H | - | - |
| 318 | Ir | 2 | 1 | L1 | B | H | H | Tn7 | H | H | H | H | H | - | - |
| | | | | L1' | B | H | H | Pe2 | H | H | H | H | - | - | - |
| 319 | Ir | 2 | 1 | L1 | B | H | H | Tn8 | H | H | H | H | H | - | - |
| | | | | L1' | B | H | H | Np4 | H | H | H | H | - | - | - |

(continued)

| No | M | m | n | L | X | R1 | R2 | X1 | X2 | X3 | X4 | R7 | R8 | R9 | R10 |
|---|---|---|---|---|---|---|---|---|---|---|---|---|---|---|---|
| | | | | L’ | X’ | R1 | R2 | X1’ | X2’ | X3’ | X4’ | R7 | R8 | R9 | R10 |
| 320 | Ir | 2 | 1 | L1 | B | H | H | Tn6 | H | H | H | H | H | - | - |
| | | | | L1’ | B | CH3 | H | H | H | H | H | - | - | - | - |
| 321 | Ir | 2 | 1 | L1 | B | CH3 | H | F | H | H | H | - | - | - | - |
| | | | | L1’ | B | CH3 | H | CF3 | H | H | H | - | - | - | - |
| 322 | Ir | 2 | 1 | L1 | B | CH3 | H | H | CF3 | H | H | - | - | - | - |
| | | | | L1’ | B | CH3 | H | F | CF3 | H | H | - | - | - | - |
| 323 | Ir | 2 | 1 | L1 | B | H | CH3 | CF3 | F | H | H | - | - | - | - |
| | | | | L1’ | B | H | CH3 | Cl | CF3 | H | H | - | - | - | - |
| 324 | Ir | 2 | 1 | L1 | B | H | CH3 | OC4H9 | H | H | H | - | - | - | - |
| | | | | L1’ | B | H | CH3 | H | OCH (CH3)2 | H | H | - | - | - | - |
| 325 | Ir | 2 | 1 | L1 | B | H | CH3 | Ph2 | H | H | H | H | F | H | H |
| | | | | L1’ | B | H | CH3 | Np3 | H | H | H | H | H | - | - |

Table 1-8

| No | M | m | n | L | X | R1 | R2 | X1 | X2 | X3 | X4 | R7 | R8 | R9 | R10 |
|---|---|---|---|---|---|---|---|---|---|---|---|---|---|---|---|
| | | | | L’ | X’ | R1 | R2 | X1’ | X2’ | X3’ | X4’ | R7 | RB | R9 | R10 |
| 326 | Ir | 2 | 1 | L1 | B | H | H | H | H | H | H | - | - | - | - |
| | | | | L1’ | C | H | - | H | H | H | H | - | - | - | - |
| 327 | Ir | 2 | 1 | L1 | B | H | H | H | H | H | H | - | - | - | - |
| | | | | L1’ | C | H | - | F | H | H | H | - | - | - | - |
| 328 | Ir | 2 | 1 | L1 | B | H | H | H | H | H | H | - | - | - | - |
| | | | | L1’ | C | H | - | F | F | H | H | - | - | - | - |
| 329 | Ir | 2 | 1 | L1 | B | H | H | H | H | H | H | - | - | - | - |
| | | | | L1’ | C | H | - | F | CF3 | H | H | - | - | - | - |
| 330 | Ir | 2 | 1 | L1 | B | H | H | H | H | H | H | - | - | - | - |
| | | | | L1’ | C | H | - | Cl | CF3 | H | H | - | - | - | - |
| 331 | Ir | 2 | 1 | L1 | B | H | H | H | H | H | H | - | - | - | - |
| | | | | | D | CH3 | H | OCH3 | H | H | H | - | - | - | - |
| 332 | Ir | 2 | 1 | L1 | B | H | H | H | H | H | H | - | - | - | - |
| | | | | L1’ | E | - | - | H | OC4H9 | H | H | - | - | - | - |
| 333 | Ir | 2 | 1 | L1 | B | H | H | H | H | H | H | - | - | - | - |
| | | | | L1’ | F | C2H5 | - | H | H | H | Cl | - | - | - | - |
| 334 | Ir | 2 | 1 | L1 | B | H | H | H | H | H | H | - | - | - | - |
| | | | | L1’ | G | H | - | Ph3 | H | H | CF3 | - | - | - | - |

(continued)

| No | M | m | n | L | X | R1 | R2 | X1 | X2 | X3 | X4 | R7 | R8 | R9 | R10 |
|---|---|---|---|---|---|---|---|---|---|---|---|---|---|---|---|
| | | | | L' | X' | R1 | R2 | X1' | X2' | X3' | X4' | R7 | RB | R9 | R10 |
| 335 | Ir | 2 | 1 | L1 | B | H | H | H | H | H | H | - | - | - | - |
| | | | | L1' | H | H | - | Ph2 | H | H | H | H | H | CF3 | H |
| 336 | Ir | 2 | 1 | L1 | B | H | H | OC4H9 | H | H | H | - | - | - | - |
| | | | | L1' | I | H | H | H | H | H | H | - | - | - | - |
| 337 | Ir | 2 | 1 | L1 | B | H | H | Br | H | H | H | - | - | - | - |
| | | | | L1' | J | H | H | CF3 | H | H | H | - | - | - | - |
| 338 | Ir | 2 | 1 | L1 | B | H | H | H | H | H | Cl | - | - | - | - |
| | | | | L1' | K | - | CH3 | Np3 | H | H | H | H | H | - | - |
| 339 | Ir | 2 | 1 | L1 | B | H | H | H | H | H | CF3 | - | - | - | - |
| | | | | L1' | M | H | - | H | F | H | H | - | - | - | - |
| 340 | Ir | 2 | 1 | L1 | B | H | H | Ph3 | H | H | CF3 | - | - | - | - |
| | | | | L1' | N | CH3 | H | H | CF3 | H | H | - | - | - | - |
| 341 | Ir | 2 | 1 | L1 | B | H | H | Ph2 | H | H | H | H | H | CF3 | H |
| | | | | L1' | O | H | H | Cl | CF3 | H | H | - | - | - | - |
| 342 | Ir | 2 | 1 | L1 | B | H | H | Np3 | H | H | H | H | H | - | - |
| | | | | L1' | P | - | H | OCH3 | H | H | H | - | - | - | - |
| 343 | Ir | 2 | 1 | L1 | B | H | H | Tn7 | H | H | H | H | H | - | - |
| | | | | L1' | Q | H | - | H | H | H | H | - | - | - | - |
| 344 | Ir | 2 | 1 | L1 | B | H | H | Tn8 | H | H | H | H | H | - | - |
| | | | | L1' | R | H | - | H | Cl | H | H | - | - | - | - |
| 345 | Ir | 2 | 1 | L1 | B | H | H | Tn6 | H | H | H | H | H | - | - |
| | | | | L1' | S | H | - | H | OC4H9 | H | H | - | - | - | - |
| 346 | Ir | 2 | 1 | L1 | B | CH3 | H | F | H | H | H | - | - | - | - |
| | | | | 1' | V | H | - | Ph2 | H | H | H | H | F | H | H |
| 347 | Ir | 2 | 1 | L1 | B | CH3 | H | H | CF3 | H | H | - | - | - | - |
| | | | | L1' | Y | H | - | Pe2 | H | H | H | H | - | - | - |
| 348 | Ir | 2 | 1 | L1 | B | H | CH3 | CF3 | F | H | H | - | - | - | - |
| | | | | L1' | A' | CH3 | - | CF3 | H | H | H | - | - | - | - |
| 349 | Ir | 2 | 1 | L1 | B | H | CH3 | OC4H9 | H | H | H | - | - | - | - |
| | | | | L1' | C' | H | H | Cl | CF3 | H | H | - | - | - | - |
| 350 | Ir | 2 | 1 | L1 | B | H | CH3 | Ph2 | H | H | H | H | F | H | H |
| | | | | L1' | D' | CH3 | - | Np3 | H | H | H | H | H | - | - |

Table 1-9

| No | M | m | n | L | X | R1 | R2 | X1 | X2 | X3 | X4 | R7 | R8 | R9 | R10 |
|---|---|---|---|---|---|---|---|---|---|---|---|---|---|---|---|
| | | | | L' | X' | R1 | R2 | X1' | X2' | X3' | X4' | R7 | R8 | R9 | R10 |
| 351 | Rh | 2 | 1 | L1 | B | H | H | H | H | H | H | - | - | - | - |
| | | | | L1' | B | H | H | F | H | H | H | - | - | - | - |
| 352 | Rh | 2 | 1 | L1 | B | H | H | H | H | H | H | - | - | - | - |
| | | | | L1' | B | H | H | H | F | H | H | - | - | - | - |
| 353 | Rh | 2 | 1 | L1 | B | H | H | H | H | H | H | - | - | - | - |
| | | | | L1' | B | H | H | F | F | H | H | - | - | - | - |
| 354 | Rh | 2 | 1 | L1 | B | H | H | H | H | H | H | - | - | - | - |
| | | | | L1' | B | H | H | CF3 | H | H | H | - | - | - | - |
| 355 | Rh | 2 | 1 | L1 | B | H | H | H | H | H | H | - | - | - | - |
| | | | | L1' | B | H | H | H | CF3 | H | H | - | - | - | - |
| 356 | Rh | 2 | 1 | L1 | B | H | H | H | H | H | H | - | - | - | - |
| | | | | L1' | B | H | H | F | CF3 | H | H | - | - | - | - |
| 357 | Rh | 2 | 1 | L1 | B | H | H | H | H | H | H | - | - | - | - |
| | | | | L1' | B | H | H | Cl | CF3 | H | H | - | - | - | - |
| 358 | Rh | 2 | 1 | L1 | B | H | H | H | H | H | H | - | - | - | - |
| | | | | L1' | B | H | H | CH3 | H | H | H | - | - | - | - |
| 359 | Rh | 2 | 1 | L1 | B | H | H | H | H | H | H | - | - | - | - |
| | | | | L1' | B | H | H | OCF3 | H | H | H | - | - | - | - |
| 360 | Rh | 2 | 1 | L1 | B | H | H | H | H | H | H | - | - | - | - |
| | | | | L1' | B | H | H | H | OC4H9 | H | H | - | - | - | - |
| 361 | Pt | 1 | 1 | L1 | B | H | H | OC4H9 | H | H | H | - | - | - | - |
| | | | | L1' | B | H | H | H | OCH (CH3)2 | H | H | - | - | - | - |
| 362 | Pt | 1 | 1 | L1 | B | H | H | Br | H | H | H | - | - | - | - |
| | | | | L1' | B | H | H | H | H | Cl | H | - | - | - | - |
| 363 | Pt | 1 | 1 | L1 | B | H | H | H | H | H | Cl | - | - | - | - |
| | | | | L1' | B | H | H | H | H | CF3 | H | - | - | - | - |
| 364 | Pt | 1 | 1 | L1 | B | H | H | H | H | H | CF3 | - | - | - | - |
| | | | | L1' | B | H | H | Ph3 | H | H | H | - | - | - | - |
| 365 | Pt | 1 | 1 | L1 | B | H | H | Ph3 | H | H | CF3 | - | - | - | - |
| | | | | L1' | B | H | H | Ph2 | H | H | H | H | F | H | H |
| 366 | Pd | 1 | 1 | L1 | B | H | H | Ph2 | H | H | H | H | H | CF3 | H |
| | | | | L1' | B | H | H | Tn5 | H | H | H | H | H | - | - |
| 367 | Pd | 1 | 1 | L1 | B | H | H | Np3 | H | H | H | H | H | - | - |
| | | | | L1' | B | H | H | H | Tn5 | H | H | H | H | - | - |

(continued)

| No | M | m | n | L | X | R1 | R2 | X1 | X2 | X3 | X4 | R7 | R8 | R9 | R10 |
|---|---|---|---|---|---|---|---|---|---|---|---|---|---|---|---|
| | | | | L' | X' | R1 | R2 | X1' | X2' | X3' | X4' | R7 | R8 | R9 | R10 |
| 368 | Pd | 1 | 1 | L1 | B | H | H | Tn7 | H | H | H | H | H | - | - |
| | | | | L1' | B | H | H | Pe2 | H | H | H | H | - | - | - |
| 369 | Pd | 1 | 1 | L1 | B | H | H | Tn8 | H | H | H | H | H | - | - |
| | | | | L1' | B | H | H | Np4 | H | H | H | H | - | - | - |
| 370 | Pd | 1 | 1 | L1 | B | H | H | Tn6 | H | H | H | H | H | - | - |
| | | | | L1' | B | CH3 | H | H | H | H | H | - | - | - | - |
| 371 | Rh | 2 | 1 | L1 | B | H | H | H | H | H | H | - | - | - | - |
| | | | | L1' | C | H | - | Cl | CF3 | H | H | - | - | - | - |
| 372 | Rh | 2 | 1 | L1 | B | H | H | Ph3 | H | H | CF3 | - | - | - | - |
| | | | | L1' | N | CH3 | H | H | CF3 | H | H | - | - | - | - |
| 373 | Pt | 1 | 1 | L1 | B | H | H | Tn6 | H | H | H | H | H | - | - |
| | | | | L1' | S | H | - | H | OC4H9 | H | H | - | - | - | - |
| 374 | Pt 1 | | 1 | L1 | B | H | CH3 | OC4H9 | H | H | H | - | - | - | - |
| | | | | L1' | C' | H | H | Cl | CF3 | H | H | - | - | - | - |
| 375 | Pd | 1 | 1 | L1 | B | H | CH3 | Ph2 | H | H | H | H | F | H | H |
| | | | | L1' | D' | CH3 | - | Np3 | H | H | H | H | H | - | - |

Table 1-10

| No | M | m | n | L | X | R1 | R2 | X1 | X2 | X3 | X4 | R7 | R8 | R9 | R10 |
|---|---|---|---|---|---|---|---|---|---|---|---|---|---|---|---|
| | | | | L' | | A" | B" | R3 | R4 | R5 | R6 | A" | | | |
| | | | | | | | | | | | | R7 | R8 | R9 | R10 |
| | | | | | | | | | | | | B" | | | |
| | | | | | | | | | | | | R7 | R8 | R9 | R10 |
| 376 | Ir | 2 | 1 | L1 | B | H | H | H | H | H | H | - | - | - | - |
| | | | | - | | Ph1 | Pi | H | H | H | H | - | - | - | - |
| | | | | | | | | | | | | - | - | - | - |
| 377 | Ir | 2 | 1 | L1 | B | H | H | F | CF3 | H | H | - | - | - | - |
| | | | | - | | Ph1 | Pi | H | H | H | H | - | - | - | - |
| | | | | | | | | | | | | - | - | - | - |

(continued)

| No | M | m | n | L | X | R1 | R2 | X1 | X2 | X3 | X4 | R7 | R8 | R9 | R10 |
|---|---|---|---|---|---|---|---|---|---|---|---|---|---|---|---|
| | | | | L' | | A" | B" | R3 | R4 | R5 | R6 | A" | | | |
| | | | | | | | | | | | | R7 | R8 | R9 | R10 |
| | | | | | | | | | | | | B" | | | |
| | | | | | | | | | | | | R7 | R8 | R9 | R10 |
| 378 | Ir | 2 | 1 | L1 | B | H | H | Cl | CF3 | H | H | - | - | - | - |
| | | | | - | | Ph1 | Pi | H | H | H | H | - | - | - | - |
| | | | | | | | | | | | | - | - | - | - |
| 379 | Ir | 2 | 1 | L1 | B | H | H | H | OCF3 | H | H | - | - | - | - |
| | | | | - | | Ph1 | Pi | H | H | H | H | - | - | - | - |
| | | | | | | | | | | | | - | - | - | - |
| 380 | Ir | 2 | 1 | L1 | B | H | H | OC4H9 | H | H | H | - | - | - | - |
| | | | | - | | Ph1 | Pi | H | H | H | H | - | - | - | - |
| | | | | | | | | | | | | - | - | - | - |
| 381 | Ir | 2 | 1 | L1 | B | H | H | Ph2 | H | H | H | H | H | CF3 | H |
| | | | | - | | Ph1 | Pi | Ph2 | H | H | H | H | F | H | H |
| | | | | | | | | | | | | - | - | - | - |
| 382 | Ir | 2 | 1 | L1 | B | H | H | Tn7 | H | H | H | H | H | - | - |
| | | | | - | | Ph1 | Pi | H | H | H | H | - | - | - | - |
| | | | | | | | | | | | | - | - | - | - |
| 383 | Ir | 2 | 1 | L1 | B | H | CH3 | Ph2 | H | H | H | H | F | H | H |
| | | | | - | | Tn2 | Pi | H | CH3 | H | H | - | - | - | - |
| | | | | | | | | | | | | - | - | - | - |
| 384 | Ir | 2 | 1 | L1 | B | H | CH3 | Np3 | H | H | H | H | H | - | - |
| | | | | - | | Tn3 | Pi | H | H | H | H | - | - | - | - |
| | | | | | | | | | | | | - | - | - | - |
| 385 | Ir | 2 | 1 | L1 | C | H | - | H | H | H | H | - | - | - | - |
| | | | | - | | Tn4 | Pi | H | H | H | H | - | - | - | - |
| | | | | | | | | | | | | - | - | - | - |
| 386 | Ir | 2 | 1 | L1 | D | H | H | CF3 | H | H | H | - | - | - | - |
| | | | | - | | Np2 | Pi | H | H | H | CF3 | - | - | - | - |
| | | | | | | | | | | | | - | - | - | - |
| 387 | Ir | 2 | 1 | L1 | E | - | - | H | Cl | H | H | - | - | - | - |
| | | | | - | | Pe1 | Py1 | H | - | H | H | - | - | - | - |
| | | | | | | | | | | | | - | - | - | - |

(continued)

| No | M | m | n | L | X | R1 | R2 | X1 | X2 | X3 | X4 | R7 | R8 | R9 | R10 |
|---|---|---|---|---|---|---|---|---|---|---|---|---|---|---|---|
| | | | | L' | | A" | B" | R3 | R4 | R5 | R6 | A" | | | |
| | | | | | | | | | | | | R7 | R8 | R9 | R10 |
| | | | | | | | | | | | | B" | | | |
| | | | | | | | | | | | | R7 | R8 | R9 | R10 |
| 388 | Ir | 2 | 1 | L1 | F | H | - | H | OCH(CH3)2 | H | H | - | - | - | - |
| | | | | - | | Tn1 | Pr | H | H | Ph3 | H | - | - | - | - |
| | | | | | | | | | | | | - | - | - | - |

Table 1-11

| No | M | m | n | L | X | R1 | R2 | X1 | X2 | X3 | X4 | R7 | R8 | R9 | R10 |
|---|---|---|---|---|---|---|---|---|---|---|---|---|---|---|---|
| | | | | L' | | A" | B" | R3 | R4 | R5 | R6 | A" | | | |
| | | | | | | | | | | | | R7 | R8 | R9 | R10 |
| | | | | | | | | | | | | B" | | | |
| | | | | | | | | | | | | R7 | R8 | R9 | R10 |
| 389 | Ir | 2 | 1 | L1 | H | H | - | Ph2 | H | H | H | H | H | CF3 | H |
| | | | | - | | Ph1 | Pi | H | H | H | Tn5 | - | - | - | - |
| | | | | | | | | | | | | H | H | - | - |
| 390 | Ir | 2 | 1 | L1 | H | CH3 | - | H | Tn5 | H | H | H | H | - | - |
| | | | | - | | Ph1 | Pi | H | H | H | Tn8 | - | - | - | - |
| | | | | | | | | | | | | H | H | - | - |
| 391 | Rh | 2 | 1 | L1 | I | H | H | Tn8 | H | H | H | H | H | - | - |
| | | | | - | | Ph1 | Pi | H | H | H | H | - | - | - | - |
| | | | | | | | | | | | | - | - | - | - |
| 392 | Rh | 2 | 1 | L1 | P | - | H | OCH3 | H | H | H | - | - | - | - |
| | | | | - | | Ph1 | Pi | H | Ph2 | H | H | F | F | F | F |
| | | | | | | | | | | | | - | - | - | - |
| 393 | Rh | 2 | 1 | L1 | V | H | - | Ph2 | H | H | H | H | F | H | H |
| | | | | - | | Tn2 | Py2 | - | H | H | H | - | - | - | - |
| | | | | | | | | | | | | - | - | - | - |
| 394 | Rh | 2 | 1 | L1 | D' | H | - | Ph2 | H | H | H | H | F | H | H |
| | | | | - | | Tn3 | Pi | Np3 | H | CF3 | H | H | H | - | - |
| | | | | | | | | | | | | - | - | - | - |

(continued)

| No | M | m | n | L | X | R1 | R2 | X1 | X2 | X3 | X4 | R7 | R8 | R9 | R10 |
|---|---|---|---|---|---|---|---|---|---|---|---|---|---|---|---|
| | | | | L' | | A" | B" | R3 | R4 | R5 | R6 | A" | | | |
| | | | | | | | | | | | | R7 | R8 | R9 | R10 |
| | | | | | | | | | | | | B" | | | |
| | | | | | | | | | | | | R7 | R8 | R9 | R10 |
| 395 | Pt | 1 | 1 | L1 | F | - | - | F | CF3 | H | H | - | - | - | - |
| | | | | - | | Ph1 | Pi | H | H | H | H | - | - | - | - |
| | | | | | | | | | | | | - | - | - | - |
| 396 | Pt | 1 | 1 | L1 | J' | - | - | Ph3 | H | H | CF3 | - | - | - | - |
| | | | | - | | Tn1 | Pi | H | H | H | H | - | - | - | - |
| | | | | | | | | | | | | - | - | - | - |
| 397 | Pt | 1 | 1 | L1 | C | H | - | Pe2 | H | H | H | H | - | - | - |
| | | | | - | | Np2 | Pi | H | H | H | H | - | - | - | - |
| | | | | | | | | | | | | - | - | - | - |
| 398 | Pd | 1 | 1 | L1 | B | H | H | H | H | H | H | - | - | - | - |
| | | | | - | | Ph1 | Pi | H | H | H | H | - | - | - | - |
| | | | | | | | | | | | | - | - | - | - |
| 399 | Pd | 1 | 1 | L1 | B | H | H | H | OCH (CH3) 2 | H | H | - | - | - | - |
| | | | | - | | Tn3 | Pi | Ph2 | H | H | CH3 | H | C3F7 | H | H |
| | | | | | | | | | | | | - | - | - | - |
| 400 | Pd | 1 | 1 | L1 | C | H | - | H | H | H | H | - | - | - | - |
| | | | | - | | Np1 | Pr | H | H | An | H | - | - | - | - |
| | | | | | | | | | | | | H | - | - | - |

Table 1-12

| No | M | m | n | L | X | R1 | R2 | X1 | X2 | X3 | X4 | R7 | R8 | R9 | R10 |
|---|---|---|---|---|---|---|---|---|---|---|---|---|---|---|---|
| | | | | E | | | | | | | | E | | | |
| | | | | | | | | | | | | R7 | R8 | R9 | R10 |
| | | | | G | | | | | | | | G | | | |
| | | | | | | | | | | | | R7 | R8 | R9 | R10 |
| 401 | Ir | 2 | 1 | L1 | B | H | H | H | H | H | H | - | - | - | - |
| | | | | CH3 | | | | | | | | - | - | - | - |
| | | | | CH3 | | | | | | | | - | - | - | - |

(continued)

| No | M | m | n | L | X | R1 | R2 | X1 | X2 | X3 | X4 | R7 | R8 | R9 | R10 |
|---|---|---|---|---|---|---|---|---|---|---|---|---|---|---|---|
| | | | | E | | | | | | | | E | | | |
| | | | | | | | | | | | | R7 | R8 | R9 | R10 |
| | | | | G | | | | | | | | G | | | |
| | | | | | | | | | | | | R7 | R8 | R9 | R10 |
| 402 | Ir | 2 | 1 | L1 | B | H | H | F | CF3 | H | H | - | - | - | - |
| 402 | Ir | 2 | 1 | CH3 | | | | | | | | - | - | - | - |
| 402 | Ir | 2 | 1 | CH3 | | | | | | | | - | - | - | - |
| 403 | Ir | 2 | 1 | L1 | B | H | H | Cl | CF3 | H | H | - | - | - | - |
| 403 | Ir | 2 | 1 | CH3 | | | | | | | | - | - | - | - |
| 403 | Ir | 2 | 1 | CH3 | | | | | | | | - | - | - | - |
| 404 | Ir | 2 | 1 | L1 | B | H | H | H | OCF3 | H | H | - | - | - | - |
| 404 | Ir | 2 | 1 | CF3 | | | | | | | | - | - | - | - |
| 404 | Ir | 2 | 1 | CF3 | | | | | | | | - | - | - | - |
| 405 | Ir | 2 | 1 | L1 | B | H | H | OC4H9 | H | H | H | - | - | - | - |
| 405 | Ir | 2 | 1 | CF3 | | | | | | | | - | - | - | - |
| 405 | Ir | 2 | 1 | CF3 | | | | | | | | - | - | - | - |
| 406 | Ir | 2 | 1 | L1 | B | H | H | Ph2 | H | H | H | H | H | CF3 | H |
| 406 | Ir | 2 | 1 | Ph3 | | | | | | | | - | - | - | - |
| 406 | Ir | 2 | 1 | Ph3 | | | | | | | | - | - | - | - |
| 407 | Ir | 2 | 1 | L1 | B | H | H | Tn7 | H | H | H | H | H | - | - |
| 407 | Ir | 2 | 1 | Ph2 | | | | | | | | H | C3H7 | H | H |
| 407 | Ir | 2 | 1 | Ph2 | | | | | | | | H | C3H7 | H | H |
| 408 | Ir | 2 | 1 | L1 | B | H | CH3 | Ph2 | H | H | H | H | F | H | H |
| 408 | Ir | 2 | 1 | Tn5 | | | | | | | | H | H | - | - |
| 408 | Ir | 2 | 1 | Tn5 | | | | | | | | H | H | - | - |
| 409 | Ir | 2 | 1 | L1 | B | H | CH3 | Np3 | H | H | H | H | H | - | - |
| 409 | Ir | 2 | 1 | CH3 | | | | | | | | - | - | - | - |
| 409 | Ir | 2 | 1 | Ph3 | | | | | | | | - | - | - | - |
| 410 | Ir | 2 | 1 | L1 | C | H | - | H | H | H | H | - | - | - | - |
| 410 | Ir | 2 | 1 | Tn6 | | | | | | | | H | H | - | - |
| 410 | Ir | 2 | 1 | Tn6 | | | | | | | | H | H | - | - |
| 411 | Ir | 2 | 1 | L1 | D | H | H | CF3 | H | H | H | - | - | - | - |
| 411 | Ir | 2 | 1 | Np3 | | | | | | | | CH30 | H | - | - |
| 411 | Ir | 2 | 1 | Np3 | | | | | | | | CH30 | H | - | - |
| 412 | Ir | 2 | 1 | L1 | E | - | - | H | Cl | H | H | - | - | - | - |
| 412 | Ir | 2 | 1 | Np4 | | | | | | | | F | - | - | - |
| 412 | Ir | 2 | 1 | Np4 | | | | | | | | F | - | - | - |

(continued)

| No | M | m | n | L | X | R1 | R2 | X1 | X2 | X3 | X4 | R7 | R8 | R9 | R10 |
|---|---|---|---|---|---|---|---|---|---|---|---|---|---|---|---|
| | | | | E | | | | | | | | E | | | |
| | | | | | | | | | | | | R7 | R8 | R9 | R10 |
| | | | | G | | | | | | | | G | | | |
| | | | | | | | | | | | | R7 | R8 | R9 | R10 |
| 413 | Ir | 2 | 1 | L1 | F | H | - | H | OCH(CH3)2 | H | H | - | - | - | - |
| | | | | Tn7 | | | | | | | | CH3 | H | - | - |
| | | | | Tn7 | | | | | | | | CH3 | H | - | - |

Table 1-13

| No | M | m | n | L | X | R1 | R2 | X1 | X2 | X3 | X4 | R7 | R8 | R9 | R10 |
|---|---|---|---|---|---|---|---|---|---|---|---|---|---|---|---|
| | | | | E | | | | | | | | E | | | |
| | | | | | | | | | | | | R7 | R8 | R9 | R10 |
| | | | | G | | | | | | | | G | | | |
| | | | | | | | | | | | | R7 | R8 | R9 | R10 |
| 414 | Ir | 2 | 1 | L1 | H | H | - | Ph2 | H | H | H | H | H | CF3 | H |
| | | | | Tn8 | | | | | | | | H | H | - | - |
| | | | | Tn8 | | | | | | | | H | H | - | - |
| 415 | Ir | 2 | 1 | L1 | H | CH3 | - | H | Tn5 | H | H | H | H | - | - |
| | | | | Pe2 | | | | | | | | H | - | - | - |
| | | | | Pe2 | | | | | | | | H | - | - | - |
| 416 | Rh | 2 | 1 | L1 | I | H | H | Tn8 | H | H | H | H | H | - | - |
| | | | | CH3 | | | | | | | | - | - | - | - |
| | | | | CH3 | | | | | | | | - | - | - | - |
| 417 | Rh | 2 | 1 | L1 | P | - | H | OCH3 | H | H | H | - | - | - | - |
| | | | | CH3 | | | | | | | | - | - | - | - |
| | | | | CH3 | | | | | | | | - | - | - | - |
| 418 | Rh | 2 | 1 | L1 | V | H | - | Ph2 | H | H | H | H | F | H | H |
| | | | | CH3 | | | | | | | | - | - | - | - |
| | | | | CH3 | | | | | | | | - | - | - | - |
| 419 | Rh | 2 | 1 | L1 | D' | H | - | Ph2 | H | H | H | H | F | H | H |
| | | | | Ph3 | | | | | | | | - | - | - | - |
| | | | | Ph3 | | | | | | | | - | - | - | - |
| 420 | Pt | 1 | 1 | L1 | F' | - | - | F | CF3 | H | H | - | - | - | - |
| | | | | CH3 | | | | | | | | - | - | - | - |
| | | | | CH3 | | | | | | | | - | - | - | - |

(continued)

| No | M | m | n | L | X | R1 | R2 | X1 | X2 | X3 | X4 | R7 | R8 | R9 | R10 |
|---|---|---|---|---|---|---|---|---|---|---|---|---|---|---|---|
| | | | | E | | | | | | | | E | | | |
| | | | | | | | | | | | | R7 | R8 | R9 | R10 |
| | | | | G | | | | | | | | G | | | |
| | | | | | | | | | | | | R7 | R8 | R9 | R10 |
| 421 | Pt | 1 | 1 | L1 | J' | - | - | Ph3 | H | H | CF3 | - | - | - | - |
| | | | | CF3 | | | | | | | | - | - | - | - |
| | | | | CF3 | | | | | | | | - | - | - | - |
| 422 | Pt | 1 | 1 | L1 | C | H | - | Pe2 | H | H | H | H | - | - | - |
| | | | | Pi2 | | | | | | | | H | H | - | - |
| | | | | Pi2 | | | | | | | | H | H | - | - |
| 423 | Pd | 1 | 1 | L1 | B | H | H | H | H | H | H | - | - | - | - |
| | | | | CH3 | | | | | | | | - | - | - | - |
| | | | | CH3 | | | | | | | | - | - | - | - |
| 424 | Pd | 1 | 1 | L1 | B | H | H | H | OCH (CH3) 2 | H | H | - | - | - | - |
| | | | | CF3 | | | | | | | | - | - | - | - |
| | | | | CF3 | | | | | | | | - | - | - | - |
| 425 | Pd | 1 | 1 | L1 | C | H | - | H | H | H | H | - | - | - | - |
| | | | | Qn2 | | | | | | | | H | H | - | - |
| | | | | Qn2 | | | | | | | | H | H | - | - |

Table 1-14

| No | M | m | n | L | X | R1 | R2 | R3 | R4 | X1 | X2 | X3 | X4 |
|---|---|---|---|---|---|---|---|---|---|---|---|---|---|
| 426 | Ir | 3 | 0 | L2 | B | H | H | - | - | H | H- | - | H |
| 427 | Ir | 3 | 0 | L3 | B | H | H | - | - | F | H | H | - |
| 428 | Ir | 3 | 0 | L4 | B | H | H | - | - | H | F | H | H |
| 429 | Ir | 3 | 0 | L5 | B | H | H | - | - | - | F | H | H |
| 430 | Ir | 3 | 0 | L6 | B | H | H | - | - | CF3 | - | - | H |
| 431 | Ir | 3 | 0 | L7 | B | H | H | - | - | H | H | H | H |
| 432 | Ir | 3 | 0 | L8 | B | H | H | - | - | F | CF3 | H | H |
| 433 | Ir | 3 | 0 | L9 | B | H | H | - | - | H | H | CF3 | F |
| 434 | Ir | 3 | 0 | L10 | B | H | H | - | - | H | H | H | H |
| 435 | Ir | 3 | 0 | L11 | B | H | H | - | - | H | H | H | H |
| 436 | Ir | 3 | 0 | L2' | B | H | H | - | - | H | CH3 | - | H |
| 437 | Ir | 3 | 0 | L3' | B | H | H | - | - | OCH3 | H | H | - |
| 438 | Ir | 3 | 0 | L4' | B | H | H | - | - | H | H | H | H |
| 439 | Ir | 3 | 0 | L5' | B | H | H | - | - | - | H | H | H |

(continued)

| No | M | m | n | L | X | R1 | R2 | R3 | R4 | X1 | X2 | X3 | X4 |
|---|---|---|---|---|---|---|---|---|---|---|---|---|---|
| 440 | Ir | 3 | 0 | L6' | B | H | H | - | - | H | - | - | H |
| 441 | Ir | 3 | 0 | L7' | B | H | H | - | - | H | H | H | H |
| 442 | Ir | 3 | 0 | L8' | B | H | H | - | - | H | H | H | H |
| 443 | Ir | 3 | 0 | L9' | B | H | H | - | - | H | H | H | H |
| 444 | Ir | 3 | 0 | L10' | B | H | H | - | - | H | H | H | H |
| 445 | Ir | 3 | 0 | L11' | B | H | H | - | - | H | H | H | H |
| 446 | Ir | 3 | 0 | L1 | M' | CH3 | CH3 | CH3 | CH3 | H | H | H | H |
| 447 | Ir | 3 | 0 | L1 | M' | C2H5 | C2H5 | C2H5 | C2H5 | H | H | H | H |
| 448 | Ir | 3 | 0 | L1 | M' | CH3 | CH3 | CH3 | CH3 | F | H | H | H |
| 449 | Ir | 3 | 0 | L1 | M' | CH3 | CH3 | CH3 | CH3 | H | F | H | H |
| 450 | Ir | 3 | 0 | L1 | M' | CH3 | CH3 | CH3 | CH3 | F | CH3 | H | H |

Hereinbelow, the present invention will be explained with reference to Examples

<Example 1> (Synthesis of Example Compound No. 1)

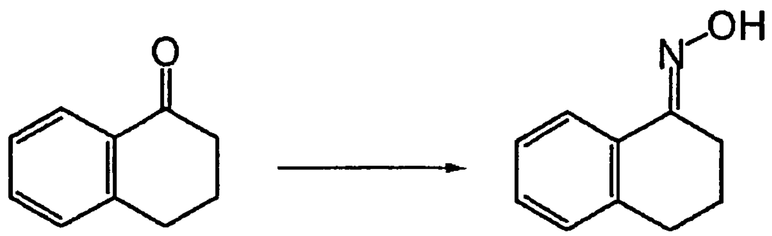

In a 2 liter-three-necked flask, 69.0 g (472 mM) of α-tetralon, 50.0 g (720 mM) of hydroxylamine hydrochloride, 500 ml of ethanol and 360 ml of 2N-sodium hydroxide aqueous solution were placed and stirred for 1 hour at room temperature. The solvent was removed under reduced pressure to obtain a residue (dry solid). To the residue, 500 ml of water was added, followed by extraction three times each with 150 ml of ethyl acetate. The organic layer was dried with anhydrous magnesium sulfate, followed by removal of the solvent under reduced pressure to obtain 74 g of a pale yellow crystal of α-tetralon = oxime (Yield: 97.2 %).

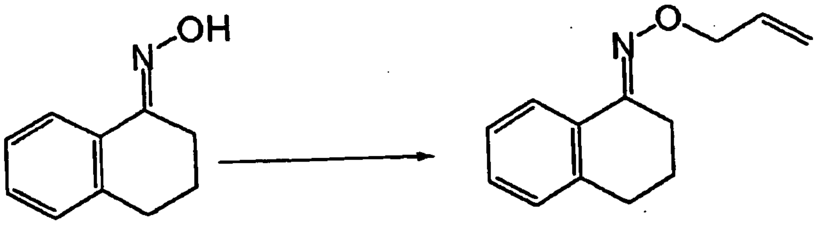

In a 1 liter-three-necked flask, 80 ml of tetrahydrofuran and 23.8 g (595 mM) of 60 %-oily sodium hydride were placed and stirred for 5 minutes at room temperature, followed by dropwise addition to a solution of 74 g (459 mM) of α-tetralon = oxime in 500 ml of anhydrous DMF (dimethylformamide) in 15 minutes. The mixture was then stirred for 1 hour at room temperature and thereto, 113.5 g (939 mM) of allyl bromide was added, followed by stirring for 12 hours

at room temperature. After the reaction, the reaction product was dried under reduced pressure to obtain a residue. To the residue, 500 ml of water was added, followed by extraction three times each with 200 ml of ethyl acetate. The organic layer was dried with anhydrous magnesium sulfate, followed by removal of the solvent under reduced pressure to obtain a brown liquid. The liquid was subjected to distillation under reduced pressure to obtain 79.5 g (Yield: 86.0 %) of α-tetralon=oxime=O-allyl=ether (boiling point = 75 = 80 °C (6.7 Pa)).

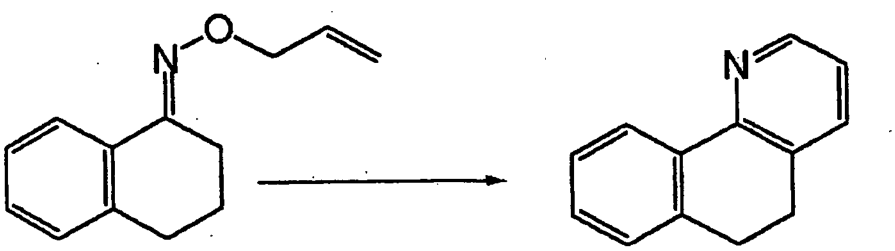

In a 1 liter-autoclave, 58.0 g (288 mM) of α-tetralon=oxime=O-allyl=ether was placed and, after being aerated with oxygen gas, sealed hermetically, followed by vigorous stirring for 5 days at 190 °C. The liquid was cooled to room temperature to obtain a high-viscous brown liquid, which was dissolved in chloroform and subjected to extraction three times each with 300 ml of 5 %-hydrochloric acid. The aqueous layer was alkalified by 48 %-sodium hydroxide and subjected to extraction three times each with 350 ml of chloroform. The organic layer was dried with anhydrous magnesium sulfate, followed by evaporation under reduced pressure. The residue was purified by silica gel column chromatography (eluent: chloroform) and further purified by silica gel column chromatography (eluent: hexane/ethyl acetate = 5/1) to obtain 7.7 g of a pale brown liquid. The liquid was purified by a Kugelroh distiller to obtain 6.6 g of colorless benzo-[h]-5,6-dihydroquinoline (Yield: 12.6 %).

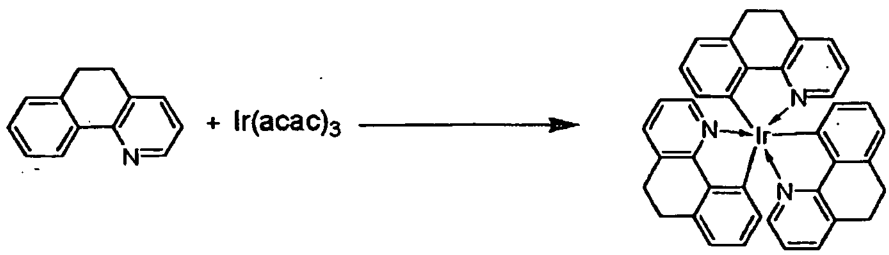


In a 100 ml-four-necked flask, 50 ml of glycerol was placed and heated for 2 hours at 130 - 140 °C under stirring and bubbling with nitrogen. The glycerol was cooled to 100 °C by standing and thereto, 0.91 g (5.02 mM) of benzo-[h]-5,6-dihydroquinoline and 0.50 g (1.02 mM) of iridium (III) acetylacetonate were placed, followed by heat-stirring for 5 hours at 190 - 215 °C in a nitrogen stream. The reaction product was cooled to room temperature and poured into 300 ml of 1N-hydrochloric acid. The precipitate was recovered by filtration, washed with water, and dissolved in acetone to remove an insoluble matter by filtration. The acetone was removed under reduced pressure to obtain a residue, which was purified by silica gel column chromatography (eluent: chloroform) to obtain 0.11 g of yellow powder of iridium (III) tris{benzo[h]-5,6-dihydroquinoline} (Yield: 14.7 %).

A solution of this compound exhibited PL (photoluminescence) spectrum having *max (maximum or peak emission wavelength) of 511 nm and quantum yield of 0.51. For comparison, when a PL spectrum of a solution of the aforementioned conventional luminescent material: $Ir(ppy)_3$ which is, not crosslinked with an alkylene group, different from the metal coordination compound, was measured, the material exhibited *manx (maximum emission wavelength) of 510 nm and a quantum yield of 0.40. Further, an organic EL device obtained in Example 3 described later produced high-luminance luminescence by electric field application. Further, EL spectrum thereof provided *max (maximum emission wavelength) of 510 nm.

<Example 2> (Synthesis of Ex. Comp. No. 53)

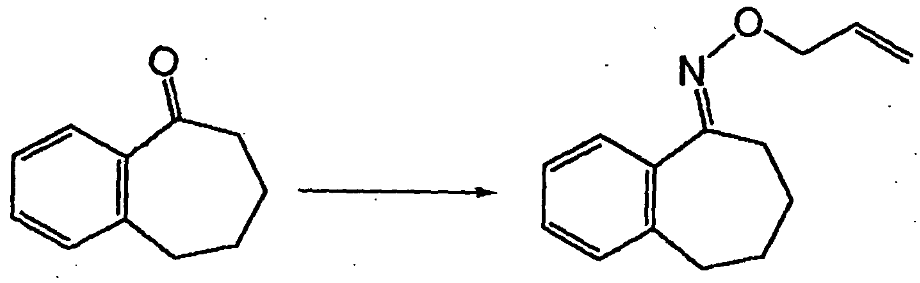

In a 3 liter-three-necked flask, 166.0 g (1036 mM) of 1-benzosuberone, 125.0 g (1141 mM) of O-allylhydroxylamine hydrochloride, 93.5 g (1140 mM) of sodium acetate, 158.0 g (1143 mM) of potassium carbonate and 1500 ml of ethanol were placed and heat-stirred for 1.5 hour at 80 °C. The reaction product was cooled to room temperature and the solvent was removed under reduced pressure to obtain a residue. To the residue, 1500 ml of water was added, followed by extraction three times each with 500 ml of ethyl acetate. The organic layer was dried with anhydrous magnesium sulfate, followed by removal of the solvent under reduced pressure to obtain a pale brown liquid. The liquid was subjected to distillation under reduced pressure to obtain 221.8 g (Yield: 99.0 %) of 1-benzosuberone=oxime=O-allyl=ether (boiling point = 75 = 83 °C (4.0 Pa)).

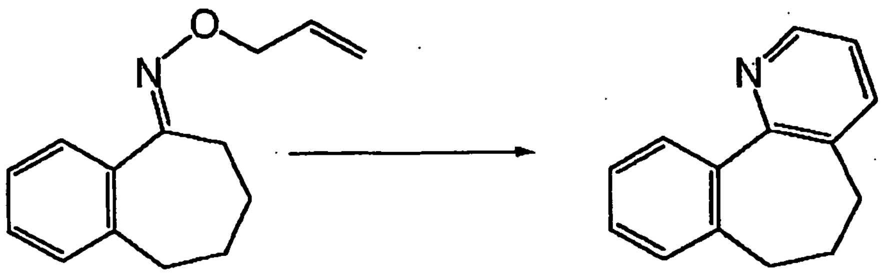

In a 5 liter-autoclave, 220.0 g (1022 mM) of 1-benzosuberone=oxime=O-allyl=ether was placed and, after being aerated with oxygen gas, sealed hermetically, followed by vigorous stirring for 3 days at 190 °C. The liquid was cooled to room temperature to obtain a high-viscous brown liquid, which was dissolved in 2 liters of chloroform and subjected to extraction three times each with 500 ml of 5 %-hydrochloric acid. The aqueous layer was alkalified by 48 %-sodium hydroxide and subjected to extraction three times each with 500 ml of chloroform. The organic layer was dried with anhydrous magnesium sulfate, followed by purification by silica gel column chromatography (eluent: hexane/ethyl acetate = 5/1) to obtain 19 g of a pale brown liquid. The liquid was purified by a Kugelroh distiller to obtain 13.5 g of pale green liquid of 3,2'-trimethylene-2-phenylpyridine (Yield: 13.5 %).

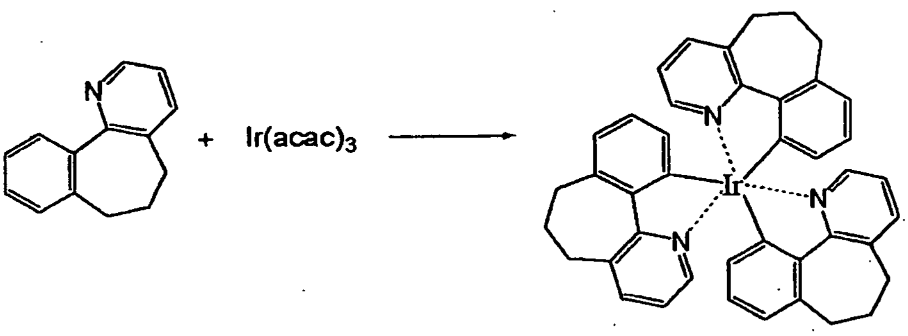


In a 100 ml-four-necked flask, 50 ml of glycerol was placed and heated for 2 hours at 130 - 140 °C under stirring and bubbling with nitrogen. The glycerol was cooled to 100 °C by standing and thereto, 0.98 g (5.02 mM) of 3,2'-trimethylene-2-phenylpyridine and 0.50 g (1.02 mM) of iridium (III) acetylacetonate were placed, followed by heat-stirring

for 8 hours at 190 - 215 °C in a nitrogen stream. The reaction product was cooled to room temperature and poured into 300 ml of 1N-hydrochloric acid. The precipitate was recovered by filtration, washed with water, and dissolved in acetone to remove an insoluble matter by filtration. The acetone was removed under reduced pressure to obtain a residue, which was purified by silica gel column chromatography (eluent: chloroform) to obtain 0.18 g of yellow powder of iridium (III) tris(3,2'-trimethylene-2-phenylpyridine) (Yield: 22.7 %).

An organic EL device obtained in Example 6 described later produced bluish green luminescence by electric field application.

<Examples 3 - 11 and Comparative Example 1>

As a device structure, a device having a three-layer structure of organic layers shown in Figure 1(b) was used. On a glass substrate (transparent substrate 15), a 100 nm-thicx film (transparent electrode 14) of ITO (indium tin oxide) was formed, followed by patterning. On the ITO-formed substrate, organic layers and metal electrode layers shown below were successively formed by vacuum (vapor) deposition using resistance heating in a vacuum chamber ($10^{-4}$ Pa).

Organic layer 1 (hole transport layer 13) (40 nm): α-NPD
Organic layer 2 (luminescence layer 12) (20 nm): mixture of CBP: luminescent material (95:5)

This layer was formed by co-deposition of CBP as a host material with a metal coordination compound shown in Table 2 below as the luminescent material so a to provide a weight proportion of 5 wt. %.

Organic layer 3 (electron transport layer 16) (30 nm): Alq3
Metal electrode layer 1 (metal electrode 11) (15 nm): Al-Li alloy (Li = 1.8 wt. %)
Metal electrode layer 2 (metal electrode 11) (100 nm): Al

After formation of the electrode material layers, patterning was performed so as to provide an electrode area of 3 $mm^2$.

An electric field (voltage) was applied, so as to provide each device with the same current value, to each luminescence device having the ITO electrode (as an anode) and the Al electrode (as a cathode), to measure a change in luminance (brightness) with time. The current amount was set to 70 $mA/cm^2$ and the respective devices showed luminances in the range of 80 - 250 $cd/m^2$ at an initial stage. A time required for decreasing these luminance values to 1/2 thereof.

For measurement, each luminescence device was taken out of the vacuum chamber and was subjected to the measurement in an atmosphere of dry nitrogen gas stream so as to remove device deterioration factors, such as oxygen and moisture (water content).

In Comparative Example 1, as the conventional luminescent material, $Ir(ppy)_3$ described in the above-mentioned Article 2 was used.

The results of continuous energization test o devices using the respective compounds are shown in Table 2. Compared with the device using the conventional luminescence material, the luminescence devices using the metal coordination compounds according to the present invention provide longer luminance half-lifes, thus resulting in a device having a high durability based on a good stability of the materials of the present invention.

Table 2

| Ex. No. | Ex.Comp. No. | Luminance half-life (Hr) |
|---|---|---|
| 3 | (1) | 950 |
| 4 | (7) | 850 |
| 5 | (48) | 700 |
| 6 | (53) | 900 |
| 7 | (102) | 600 |
| 8 | (131) | 500 |
| 9 | (302) | 800 |
| 10 | (376) | 750 |
| 11 | (401) | 650 |
| Comp.Ex. 1 | $Ir(ppy)_3$ | 350 |

<Example 12>

With reference to Figure 2, an embodiment such that the electroluminescence device of the present invention is applied to an active matrix-type color organic EL display using a TFT circuit shown in Figure 3 will be described.

Figure 2 schematically illustrates an example of a panel structure provided with an organic EL device and drive means. In this embodiment, the number of pixels was 128x128 pixels. Incidentally, one pixel was constituted by three color pixels consisting of green pixel, blue pixel and red pixel.

On a glass substrate, a thin film transistor circuit (referred to as a "TFT circuit") using polysilicon was formed in a known manner.

In a region corresponding to each of the color pixels, by using a hard mask, organic layers and metal electrode layers were formed by vacuum deposition in the thicknesses indicated below, followed by patterning. Structures of the organic layers corresponding to the respective pixels are shown below.

Green pixel:α-NPD (40 nm)%CBP:phosphorescent material (= 93:7 in weight ratio) (30 nm)/BCP (20 nm)/Alq (40 nm)
Blue pixel:α-NPD (50 nm)/BCP (20 nm)/alq (50 nm)
Red pixel:α-NPD (40 nm)/CBP:PtOEP (= 93:7 in weight ratio) (30 nm)/BCP (20 nm)/Alq (40 nm)

The luminescence layer for the green pixel was formed by co-deposition of CBP as a host material with the phosphorescent material (Ex. Comp. No. 1) which provided a weight proportion of 7 %.

In the panel shown in Figure 2, a scanning signal driver, data signal driver any a current supply source and disposed and are respectively connected to gate selection lines, data signal lines and current supply lines. At intersections of the gate selection lines and the data signal lines, a pixel circuit (equivalent circuit) shown in Figure 3 is disposed. The scanning signal driver sequentially selects gate scanning lines G1, G2, G3, ..., Gn, and in synchronism therewith, picture signals are supplied from the data signal driver.

Next, a pixel circuit operation is described with reference to the equivalent circuit shown in Figure 3. When a selection signal is applied to a gate selection line, TFT1 is turned on so that a display signal is supplied from a data signal line to a capacitor Cadd, thereby determining the gate potential of TFT2. A current is supplied to an organic luminescence device portion (abbreviated as EL) disposed at each pixel through a current supply line depending on the gate potential of TFT2. The gate potential of TFT2 is held at Cadd during one frame period, so that the current continually flows from the current supply line to the EL device portion during the period. As a result, luminescence is retained during one frame period.

As a result, it has been confirmed that desired picture information can be displayed, and it has been found that good images are stably displayed.

In this embodiment, although the driving scheme using the TFT circuit being the active matrix scheme was used as the application to the display, the switching device used in the present invention need not be particularly restricted, and even a singler crystal silicon substrate, an MIM (metal-insulator-metal) device, an a-Si (amorphous silicon) TFT circuit, etc., can be readily applied thereto.

[INDUSTRIAL APPLICABILITY]

As described above, a luminescence device using, as a luminescence material, the metal coordination compound represented by the aforementioned formula (1) of the present invention could provide a high luminescence efficiency and retain a high-brighthess luminescence or a long period of time. Further, the material is an excellent material capable of adjusting an emission wavelength, particularly providing a shorter wavelength. The luminescence device of the present invention is also excellent as a display device.

The high-efficiency luminescence device shown in the present invention is applicable to products requiring energy saving and high brightness. As applied examples, applications to a display apparatus, an illumination apparatus, a light source of a printer and a backlight for a liquid crystal display apparatus may be exemplified. As the display apparatus, it is possible to provide a flat panel display allowing energy saving, high recognizability or weight reduction. Further, as the light source or the printer, it is possible to replace a laser light source portion of a laser beam printer which has been currently used widely, with the luminescence device of the present invention. Image formation is performed by arranging independently addressable devices in the form of an array and effecting desired exposure to light against a photosensitive drum. By using the device of the present invention, it is possible to remarkably reduce an apparatus volume (size).

**Claims**

1. A metal coordination compound represented by formula (1) shown below:

$$ML_mL'_n \qquad (1)$$

wherein M is a metal atom of Ir; L and L' are mutually different bidentate ligands; m is 2 or 3; n is 0, 1 or 2 with the proviso that m+n is 3; a partial structure MLm is represented by formula (2) shown below and a partial structure ML'n is represented by formula (3), (4) or (5) shown below:

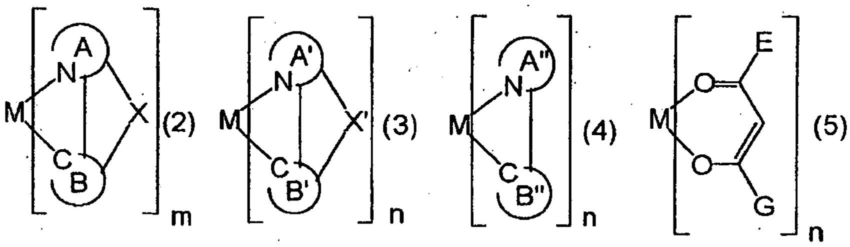

(2) (3) (4) (5)

wherein N and C are nitrogen and carbon atoms, respectively; A and A' are respectively a cyclic group capable of having a substituent and bonded to the metal atom M via the nitrogen atom; B, B' and B" are respectively a cyclic group capable of having a substituent and connected to the metal atom M via the carbon atom;
{wherein the substituent is selected from a halogen atom, a cyano group, a nitro group, a trialkylsilyl group (of which the alkyl groups are independently a linear or branched alkyl group having 1 to 8 carbon atoms), a linear or branched alkyl group having 1 to 20 carbon atoms (of which the alkyl group can include one or non-neighboring two or more methylene groups that can be replaced with -0-, -S-, - CO-, -CO-O-, -O-CO-, -CH=CH- or -C≡C- and the alkyl group can include a hydrogen atom that can be replaced with a fluorine atom), or an aromatic cyclic group capable of having a substituent (of which the substituent is a halogen atom, a cyano group, a nitro group, or a linear or branched alkyl group having 1 to 20 carbon atoms (of which the alkyl group can include one or non-neighboring two or more methylene groups that can be replaced with -O-, -S-, -CO-, -CO-O-, - O-CO-, -CH=CH- or -C≡C- and the alkyl group can include a hydrogen atom that can be replaced with a fluorine atom));
A and B, A' and B', and A" and B" are respectively bonded to each other via a covalent bond; and A and B, and A' and B' are respectively bonded to each other via X and X', respectively;
X and X' are independently a linear or branched alkylene group having 2 to 10 carbon atoms (of which the alkylene group can include one or non-neighboring two or more methylene groups that can be replaced with -O-, -S-, - CO-, -CO-O-, -O-CO-, -CH=CH- or -C≡C- and the alkylene group can include a hydrogen atom that can be replaced with a fluorine atom);
E and G are independently a linear or branched alkyl group having 1 to 20 carbon atoms (of which the alkyl group can include a hydrogen atom that can be optionally replaced with a fluorine atom), or an aromatic cyclic group capable of having a substituent (of which the substituent is a halogen atom, a cyano group, a nitro group, a trialkylsilyl group (of which the alkyl groups are independently a linear or branched alkyl group having 1 to 8 carbon atoms), or a linear or branched alkyl group having 1 to 20 carbon atoms (of which the alkyl group can include one or non-neighboring two or more methylene groups that can be replaced with -O-, -S-, -CO-, -CO-O-, -O-CO-, -CH=CH- or - C≡C- and the alkyl group can include a hydrogen atom that can be replaced with a fluorine atom)},
wherein the metal coordination compound produces green phosphorescence.

2. A compound according to Claim 1, wherein n is 0 in the formula 1).

3. A compound according to Claim 1, wherein in the formula (1), the partial structure ML'n is represented by the formula (3).

4. A compound according to Claim 1, wherein in the formula (1), the partial structure ML'n is represented by the formula (4).

5. A compound according to Claim 1, wherein in the formula (1), the partial structure ML'n is represented by the formula (5).

6. A compound according to Claim 1, wherein X is a linear or branched alkylene group having 2 to 10 carbon atoms (of which the alkylene group can include one or non-neighboring two or more methylene groups that can be replaced with -O-, -S-, -CO-, -CO-O-, -O-CO-, -CH=CH- or - C≡-C- and the alkylene group can include a hydrogen atom that can be replaced with a fluorine atom).

7. An electroluminescence device, comprising: a substrate, a pair of electrodes disposed on the substrate, and at least one species of a metal coordination compound, represented by the formula (1) according to Claim 1, disposed between the pair of electrodes.

8. A device according to Claim 7, wherein a voltage is applied between the electrodes to produce phosphorescence.

9. A compound according to Claim 1, wherein the metal coordination compound is represented by the following formula:

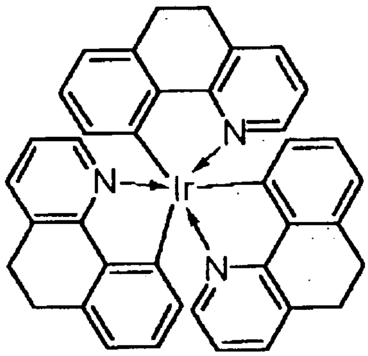

.

10. A compound according to Claim 1, wherein the metal coordination compound is represented by the following formula:

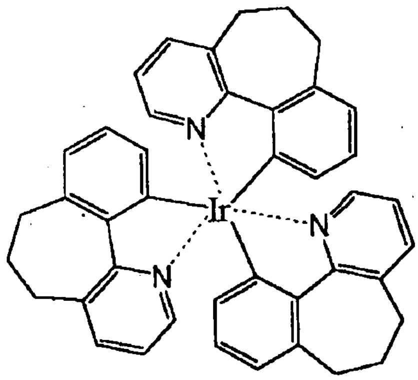

.

## Patentansprüche

1. Metallkoordinationsverbindung, dargestellt durch nachfolgend gezeigte Formel (1):

$ML_mL'_n$ (1),

wobei M ist ein Metallatom von Ir; L und L' sind gegenseitig verschiedene zweizähnige Liganden; m ist 2 oder 3; n ist 0, 1 oder 2, mit der Maßgabe, dass m+n ist 3; eine Teilstruktur MLm durch die nachfolgend gezeigte Formel (2) dargestellt ist und eine Teilstruktur ML'n durch die nachfolgend gezeigte Formel (3), (4) oder (5)

dargestellt ist:

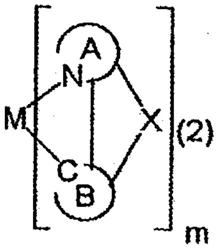 (2) 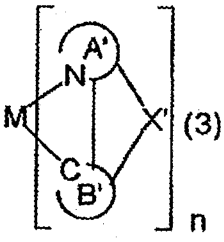 (3) 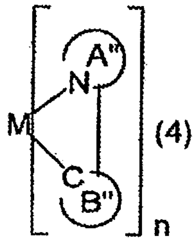 (4) 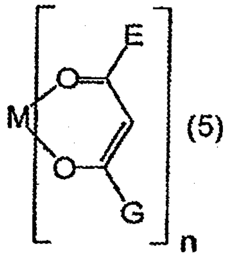 (5)

wobei N und C sind Stickstoff-, beziehungsweise Kohlenstoffatome; A und A' sind jeweils eine cyclische Gruppe, die fähig ist, einen Substituenten aufzuweisen und durch das Stickstoffatom am Metallatom M gebunden ist; B, B' und B " sind jeweils eine cyclische Gruppe, die fähig ist, einen Substituenten aufzuweisen und durch das Kohlenstoff mit dem Metallatom M verbunden ist;

{wobei der Substituent ausgewählt ist aus einem Halogenatom, einer Cyanogruppe, einer Nitrogruppe, einer Trialkylsilylgruppe (wovon die Alkylgruppen unabhängig eine lineare oder verzweigte Alkylgruppe mit 1 bis 8 Kohlenstoffatomen sind), einer linearen oder verzweigten Alkylgruppe mit 1 bis 20 Kohlenstoffatomen (wovon die Alkylgruppe eine oder nicht-benachbarte zwei oder mehr Methylengruppen beinhalten kann, die durch -O-, -S-, -CO-, -CO-O-, -O-CO-. -CH=CH- oder -C≡C- ersetzt werden können, und die Alkylgruppe ein Wasserstoffatom beinhalten kann, das durch ein Fluoratom ersetzt werden kann), oder einer aromatischen cyclischen Gruppe, die fähig ist, einen Substituenten aufzuweisen (wovon der Substituent ein Halogenatom, eine Cyanogruppe, eine Nitrogruppe, oder eine lineare oder verzweigte Alkylgruppe mit 1 bis 20 Kohlenstoffatomen ist (wovon die Alkylgruppe eine oder nicht-benachbarte zwei oder mehr Methylengruppen beinhalten kann, die durch -O-, -S-, -CO-, -CO-O-, -O-CO-. -CH=CH- oder -C≡C- ersetzt werden können, und die Alkylgruppe ein Wasserstoffatom beinhalten kann, das durch ein Fluoratom ersetzt werden kann));

A und B, A' und B', und A" und B" jeweils durch eine kovalente Bindung miteinander verbunden sind; und A und B, und A' und B' jeweils durch X, beziehungsweise X' miteinander verbunden sind;

X und X' unabhängig eine lineare oder verzweigte Alkylengruppe mit 2 bis 10 Kohlenstoffatomen sind (wovon die Alkylengruppe eine oder nicht-benachbarte zwei oder mehr Methylengruppen beinhalten kann, die durch -O-, -S-, -CO-, -CO-O-, -O-CO-. -CH=CH- oder -C≡C- ersetzt werden können, und die Alkylengruppe ein Wasserstoffatom beinhalten kann, das durch ein Fluoratom ersetzt werden kann);

E und G sind unabhängig eine lineare oder verzweigte Alkylgruppe mit 1 bis 20 Kohlenstoffatomen (wovon die Alkylgruppe ein Wasserstoffatom beinhalten kann, das optional durch ein Fluoratom ersetzt werden kann), oder eine aromatische cyclische Gruppe, die fähig ist, einen Substituenten aufzuweisen (wovon der Substituent ist ein Halogenatom, eine Cyanogruppe, eine Nitrogruppe, eine Trialkylsilylgruppe (wovon die Alkylgruppen unabhängig eine lineare oder verzweigte Alkylgruppe mit 1 bis 8 Kohlenstoffatomen sind), oder eine lineare oder verzweigte Alkylgruppe mit 1 bis 20 Kohlenstoffatomen (wovon die Alkylgruppe eine oder nicht-benachbarte zwei oder mehr Methylengruppen beinhalten kann, die durch -O-, -S-, -CO-, -CO-O-, -O-CO-, -CH=CH- oder -C≡C- ersetzt werden können, und die Alkylgruppe ein Wasserstoffatom beinhalten kann, das durch ein Fluoratom ersetzt werden kann)},

wobei die Metallkoordinationsverbindung grüne Phosphoreszenz erzeugt.

**2.** Verbindung nach Anspruch 1, wobei n in der Formel (1) 0 ist.

**3.** Verbindung nach Anspruch 1, wobei in der Formel (1) die Teilstruktur ML'n durch die Formel (3) dargestellt ist.

**4.** Verbindung nach Anspruch 1, wobei in der Formel (1) die Teilstruktur ML'n durch die Formel (4) dargestellt ist.

**5.** Verbindung nach Anspruch 1, wobei in der Formel (1) die Teilstruktur ML'n durch die Formel (5) dargestellt ist.

**6.** Verbindung nach Anspruch 1, wobei X eine lineare oder verzweigte Alkylengruppe mit 2 bis 10 Kohlenstoffatomen ist (wovon die Alkylengruppe eine oder nicht-benachbarte zwei oder mehr Methylengruppen beinhalten kann, die durch -O-, -S-, -CO-, -CO-O-, -O-CO-, -CH=CH- oder -C≡C- ersetzt werden können, und die Alkylengruppe ein Wasserstoffatom beinhalten kann, das durch ein Fluoratom ersetzt werden kann).

7. Elektrolumineszenzeinrichtung, umfassend: ein Substrat, ein auf dem Substrat angeordnetes Elektrodenpaar, und wenigstens eine Spezies einer durch die Formel (1) nach Anspruch 1 dargestellten Metallkoordinationsverbindung, die zwischen dem Elektrodenpaar angeordnet ist.

8. Vorrichtung nach Anspruch 7, wobei eine Spannung zwischen den Elektroden angelegt ist, um Phosphoreszenz zu erzeugen.

9. Verbindung nach Anspruch 1, wobei die Metallkoordinationsverbindung durch die nachfolgende Formel dargestellt ist:

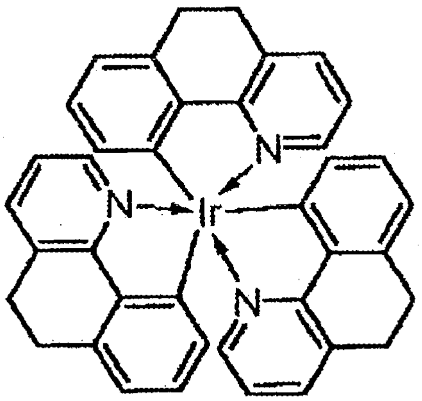

10. Verbindung nach Anspruch 1, wobei die Metallkoordinationsverbindung durch die nachfolgende Formel dargestellt ist:

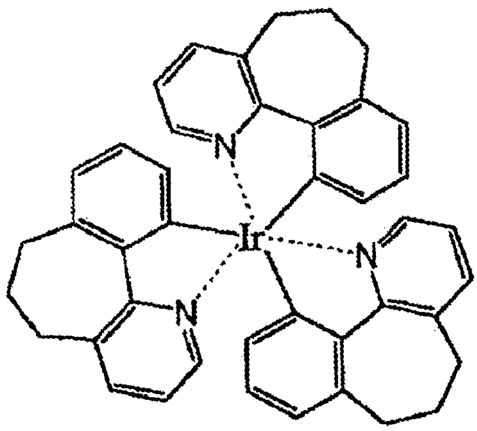

**Revendications**

1. Composé de coordination métallique représenté par la formule (1) montrée ci-dessous .

$$ML_mL'_n \qquad (1)$$

où M est un atome métallique de Ir ; L et L' sont des ligands bidentés mutuellement différents ; m est 2 ou 3 ; n est 0, 1 ou 2, à condition que m+n soit 3 ; une structure partielle MLm est représentée par la formule (2) montrée ci-dessous et une structure partielle ML'n est représentée par la formule (3), (4) ou (5) montrée ci-dessous :

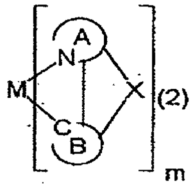 (2) 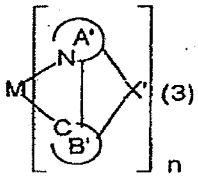 (3) 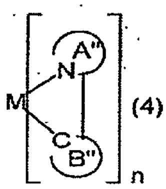 (4) 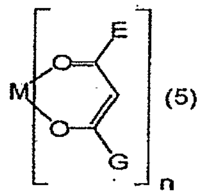 (5)

où N et C sont respectivement des atomes d'azote et de carbone ; A et A' sont respectivement un groupe cyclique capable d'avoir un substituant et lié à l'atome métallique M au moyen de l'atome d'azote ; B, B' et B" sont respectivement un groupe cyclique capable d'avoir un substituant et lié à l'atome métallique M au moyen de l'atome de carbone ;

{où le substituant est choisi parmi un atome d'halogène, un groupe cyano, un groupe nitro, un groupe trialkylsilyle (dont les groupes alkyle sont indépendamment un groupe alkyle linéaire ou ramifié ayant 1 à 8 atomes de carbone), un groupe alkyle linéaire ou ramifié ayant 1 à 20 atomes de carbone (ce groupe alkyle pouvant comprendre un ou bien deux ou plusieurs groupes méthylène non contigus qui peuvent être remplacés par -O-, -S-, -CO-, -CO-O-, -O-CO-, -CH=CH- ou -C≡C- et ce groupe alkyle pouvant comprendre un atome d'hydrogène qui peut être remplacé par un atome de fluor), ou un groupe cyclique aromatique capable d'avoir un substituant (dont le substituant est un atome d'halogène, un groupe cyano, un groupe nitro ou un groupe alkyle linéaire ou ramifié ayant 1 à 20 atomes de carbone (ce groupe alkyle pouvant comprendre un ou bien deux ou plusieurs groupes méthylène non contigus qui peuvent être remplacés par -O-, -S-, -CO-, -CO-O-, -O-CO-, -CH=CH- ou -C≡C- et ce groupe alkyle pouvant comprendre un atome d'hydrogène qui peut être remplacé par un atome de fluor)) ;

A et B, A' et B', et A" et B" sont respectivement liés l'un à l'autre par une liaison covalente ; et A et B, et A' et B' sont respectivement liés l'un à l'autre respectivement par X et X' ;

X et X' sont indépendamment un groupe alkylène linéaire ou ramifié ayant 2 à 10 atomes de carbone (ce groupe alkylène pouvant comprendre un ou bien deux ou plusieurs groupes méthylène non contigus qui peuvent être remplacés par -O-, -S-, -CO-, -CO-O-, -O-CO-, -CH=CH- ou -C≡C- et ce groupe alkylène pouvant comprendre un atome d'hydrogène qui peut être remplacé par un atome de fluor) ;

E et G sont indépendamment un groupe alkyle linéaire ou ramifié ayant 1 à 20 atomes de carbone (ce groupe alkyle pouvant comprendre un atome d'hydrogène qui peut facultativement être remplacé par un atome de fluor), ou un groupe cyclique aromatique capable d'avoir un substituant (dont le substituant est un atome d'halogène, un groupe cyano, un groupe nitro, un groupe trialkylsilyle (dont les groupes alkyle sont indépendamment un groupe alkyle linéaire ou ramifié ayant 1 à 8 atomes de carbone) ou un groupe alkyle linéaire ou ramifié ayant 1 à 20 atomes de carbone (ce groupe alkyle pouvant comprendre un ou bien deux ou plusieurs groupes méthylène non contigus qui peuvent être remplacés par -O-, -S-, -CO-, -CO-O-, O-CO-, -CH=CH- ou -C≡C- et ce groupe alkyle pouvant comprendre un atome d'hydrogène qui peut être remplacé par un atome de fluor))

dans lequel le composé de coordination métallique produit une phosphorescence verte.

2. Composé selon la revendication 1, dans lequel n est 0 dans la formule (1).

3. Composé selon la revendication 1, dans lequel, dans la formule (1), la structure partielle ML'n est représentée par la formule (3).

4. Composé selon la revendication 1, dans lequel, dans la formule (1), la structure partielle ML'n est représentée par la formule (4).

5. Composé selon la revendication 1, dans lequel, dans la formule (1), la structure partielle ML'n est représentée par la formule (5).

6. Composé selon la revendication 1, dans lequel X est un groupe alkylène linéaire ou ramifié ayant 2 à 10 atomes de carbone (ce groupe alkylène pouvant comprendre un ou bien deux ou plusieurs groupes méthylène non contigus qui peuvent être remplacés par -O-, -S-, -CO-, -CO-O-, -O-CO-, -CH=CH- ou -C≡C- et ce groupe alkylène pouvant comprendre un atome d'hydrogène qui peut être remplacé par un atome de fluor).

7. Dispositif électroluminescent comprenant : un substrat, deux électrodes disposées sur le substrat, et au moins une

espèce d'un composé de coordination métallique représenté par la formule (1) selon la revendication 1, disposée entre les deux électrodes.

**8.** Dispositif selon la revendication 7, dans lequel une tension est appliquée entre les électrodes pour produire une phosphorescence.

**9.** Composé selon la revendication 1, dans lequel le composé de coordination métallique est représenté par la formule suivante :

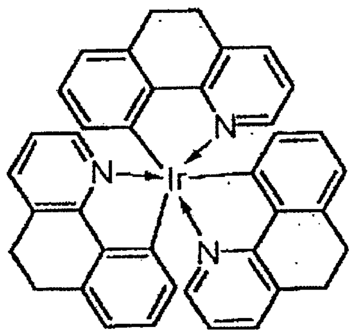

**10.** Composé selon la revendication 1, dans lequel le composé de coordination métallique est représenté par la formule suivante :

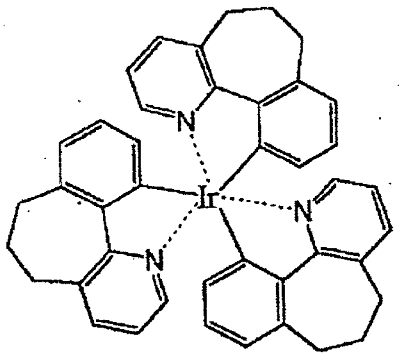

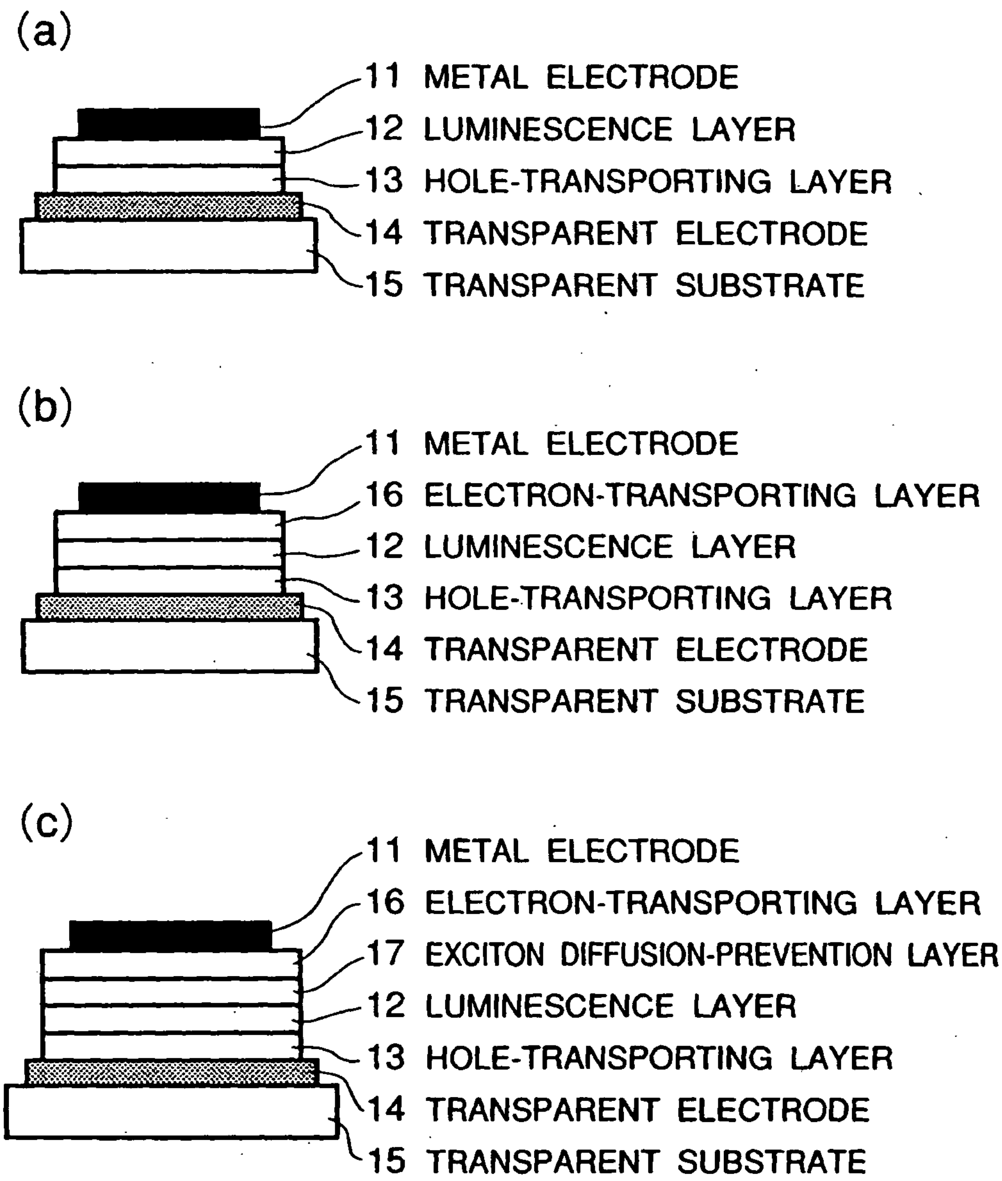
(a)
11 METAL ELECTRODE
12 LUMINESCENCE LAYER
13 HOLE-TRANSPORTING LAYER
14 TRANSPARENT ELECTRODE
15 TRANSPARENT SUBSTRATE
(b)
11 METAL ELECTRODE
16 ELECTRON-TRANSPORTING LAYER
12 LUMINESCENCE LAYER
13 HOLE-TRANSPORTING LAYER
14 TRANSPARENT ELECTRODE
15 TRANSPARENT SUBSTRATE
(c)
11 METAL ELECTRODE
16 ELECTRON-TRANSPORTING LAYER
17 EXCITON DIFFUSION-PREVENTION LAYER
12 LUMINESCENCE LAYER
13 HOLE-TRANSPORTING LAYER
14 TRANSPARENT ELECTRODE
15 TRANSPARENT SUBSTRATE

FIG. 1

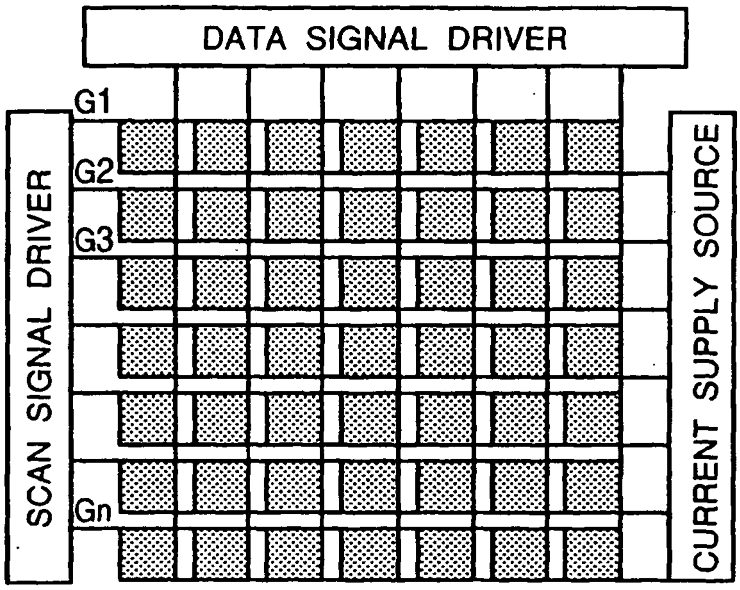
DATA SIGNAL DRIVER
G1
G2
G3
Gn
SCAN SIGNAL DRIVER
CURRENT SUPPLY SOURCE

FIG. 2

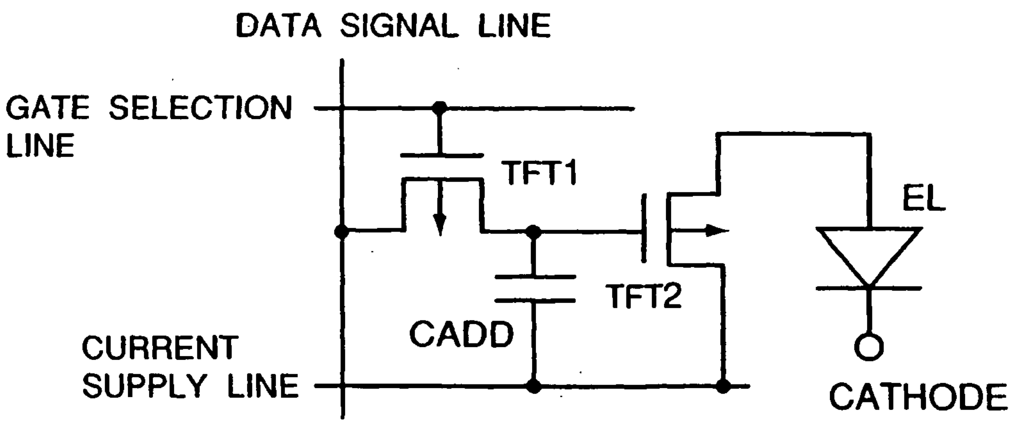
DATA SIGNAL LINE
GATE SELECTION LINE
TFT1
EL
TFT2
CADD
CURRENT SUPPLY LINE
CATHODE

FIG. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0057676 A
- JP 2001181617 A


### Non-patent literature cited in the description


- *Macromol Symp.,* 1977, vol. 125, 1-48
- **D.F. O'BRIEN et al.** *Applied Physics Letters,* 1999, vol. 74 (3), 422
- **M.A. BALDO et al.** *Applied Physics Letters,* 1999, vol. 75 (1), 4
- **LAMANSKY et al.** Synthesis and Characterization of Phosphorescent Cyclometalated Iridium Complexes. *Inorg. Chem.,* 2001, vol. 40, 1704-1711
- Highly Phosphorescent Bis-Cyclometalated Iridium Complexes: Synthesis, Photophysical Characterization, and Use in Organic Light Emitting Diodes. *J. Am. Chem. Soc.,* 2001, vol. 123, 4303-4312
- **JOLLIET et al.** Cyclometalated Complexes of Palladium(II) and Platinum(II): cis-Configured Homoleptic and Heteroleptic Compounds with Aromatic C□N Ligands. *Inorg. Chem.,* 1996, vol. 35, 4883-4888
- Electrochemistry and Spectroscopy of Ortho-Metalated Complexes of Ir(III) and Rh(III. *J. Phys. Chem.,* 1987, vol. 91, 1047-1054